(19) Europäisches Patentamt
European Patent Office
Office européen des brevets

(11) **EP 3 028 202 B1**

(12) **FASCICULE DE BREVET EUROPEEN**

(45) Date de publication et mention de la délivrance du brevet:
**13.09.2017 Bulletin 2017/37**

(21) Numéro de dépôt: **14750588.7**

(22) Date de dépôt: **28.07.2014**

(51) Int Cl.:
***G06F 19/24*** *(2011.01)*

(86) Numéro de dépôt international:
**PCT/FR2014/051952**

(87) Numéro de publication internationale:
**WO 2015/015106 (05.02.2015 Gazette 2015/05)**

(54) **PROCÉDÉ ET DISPOSITIF D'ANALYSE D'UN ÉCHANTILLON BIOLOGIQUE**

VERFAHREN UND VORRICHTUNG ZUR ANALYSE EINER BIOLOGISCHEN PROBE

METHOD AND DEVICE FOR ANALYSING A BIOLOGICAL SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.07.2013 FR 1357614**

(43) Date de publication de la demande:
**08.06.2016 Bulletin 2016/23**

(73) Titulaire: **Biomérieux S.A.**
**69280 Marcy L'Etoile (FR)**

(72) Inventeurs:
• **MAHE, Pierre**
  **F-38250 Lans en Vercors (FR)**
• **VEYRIERAS, Jean-Baptiste**
  **F-69007 Lyon (FR)**

(74) Mandataire: **Le Mauff, Frédéric Francis Joseph**
**bioMérieux**
**376, chemin de l'Orme**
**69280 Marcy-l'Étoile (FR)**

(56) Documents cités:
**EP-A1- 2 600 385**

• **KATRIEN DE BRUYNE ET AL: "Bacterial species identification from MALDI-TOF mass spectra through data analysis and machine learning", SYSTEMATIC AND APPLIED MICROBIOLOGY, vol. 34, no. 1, 1 janvier 2011 (2011-01-01), pages 20-29, XP028145446, ISSN: 0723-2020, DOI: 10.1016/J.SYAPM.2010.11.003 [extrait le 2010-12-24]**
• **T. VILLMANN ET AL: "Classification of mass-spectrometric data in clinical proteomics using learning vector quantization methods", BRIEFINGS IN BIOINFORMATICS, vol. 9, no. 2, 1 janvier 2007 (2007-01-01), pages 129-143, XP055054943, ISSN: 1467-5463, DOI: 10.1093/bib/bbn009**
• **M. S. LINDNER ET AL: "Metagenomic abundance estimation and diagnostic testing on species level", NUCLEIC ACIDS RESEARCH, vol. 41, no. 1, 31 août 2012 (2012-08-31), pages e10, p1-e10, p8, XP055113563, ISSN: 0305-1048, DOI: 10.1093/nar/gks803**
• **Pierre Mahé ET AL: "Automatic identification of mixed bacterial species fingerprints in a MALDI-TOF mass-spectrum", , 17 janvier 2014 (2014-01-17), pages 1-7, XP055113528, Extrait de l'Internet: URL:http://bioinformatics.oxfordjournals.org/content/early/2014/01/17/bioinformatics .btu022.full.pdf [extrait le 2014-04-10]**

EP 3 028 202 B1

## Description

### DOMAINE DE L'INVENTION

L'invention a trait au domaine de l'analyse d'échantillons biologiques pouvant comporter plusieurs microorganismes différents, et plus particulièrement la détection et l'identification de mélanges microbiens, à partir de techniques de mesure produisant un signal numérique multidimensionnel représentatif de l'échantillon biologique objet de l'analyse.

### ETAT DE LA TECHNIQUE

Il est connu d'utiliser la spectrométrie ou la spectroscopie pour identifier des micro-organismes, et plus particulièrement des bactéries. Pour ce faire, un échantillon d'un micro-organisme inconnu à identifier est préparé puis un spectre de masse, vibrationnel ou de fluorescence de l'échantillon est acquis et pré-traité, notamment pour éliminer la ligne de base (communément appelé « baseline ») et pour éliminer le bruit. Le spectre pré-traité est alors « comparé » à l'aide d'outil de classification à une base de référence construite à partir d'un ensemble de spectres associés à des taxons de microorganismes identifiés, par exemple des espèces, par une méthode de référence.

Plus particulièrement, l'identification de microorganismes par classification consiste classiquement en une première étape de détermination, à l'aide d'un apprentissage supervisé, d'un modèle de classification en fonction de spectres dits « d'apprentissage » de micro-organismes dont on connaît au préalable les espèces, le modèle de classification définissant un ensemble de règles distinguant ces différentes espèces parmi les spectres d'apprentissage, et en une seconde étape d'identification, ou de « prédiction », d'un microorganisme particulier inconnu. Cette seconde étape consiste notamment à faire l'acquisition d'un spectre du microorganisme à identifier, à pré-traiter le spectre et à appliquer au spectre pré-traité un modèle de prédiction construit à partir du modèle de classification afin de déterminer au moins une espèce auquel le microorganisme inconnu appartient.

Typiquement, un appareil d'identification par spectrométrie ou spectroscopie comporte ainsi un spectromètre ou spectroscope et une unité d'acquisition et de traitement recevant les spectres mesurés, numérisant ces derniers de manière à obtenir un vecteur d'intensité numérique multidimensionnel et mettant en oeuvre la seconde étape précitée en fonction du vecteur numérique produit. La première étape est quant à elle mise en oeuvre par le constructeur de l'appareil qui détermine le modèle de classification et le modèle de prédiction et l'intègre dans la machine avant son exploitation par un client.

Jusqu'à présent, quelle que soit la technique de mesure ou l'algorithme d'identification considéré, l'analyse d'un échantillon biologique est restreinte à des échantillons comportant un seul et unique type de microorganisme. En effet, l'analyse d'échantillons biologiques comportant une pluralité de microorganismes différents est particulièrement difficile et on observe notamment que les algorithmes de prédiction se basant sur des modèles de classification échouent à détecter qu'un échantillon biologique comporte plusieurs microorganismes, et donc également à identifier les microorganismes contenus dans un tel échantillon.

Ainsi, préalablement à toute étape d'identification par spectrométrie ou spectroscopie, un prélèvement à tester, dont on cherche à connaître les microorganismes qu'il contient, subit tout d'abord une étape de traitement biologique visant à isoler les différents types de microorganismes. Un échantillon biologique objet d'une identification par spectrométrie ou spectroscopie est ensuite préparé à partir d'un seul type de microorganisme isolé. Par exemple, s'agissant de l'identification de bactéries, une solution du produit à tester est préparée, puis la solution obtenue est mise en présence d'un ou plusieurs milieux de culture, par exemple sur une ou plusieurs boites de Pétri. Après incubation, différentes colonies bactériennes sont alors identifiées et isolées, chacune d'entre elles pouvant faire l'objet d'une identification ultérieure.

Or, une telle préparation d'échantillon biologique peut prendre beaucoup de temps, certains types de microorganismes nécessitant en effet des durées d'incubation de plusieurs jours. En outre, certains microorganismes nécessitent un milieu de culture très spécifique pour croître.

Outre le coût que cela engage, il existe toujours un risque de ne pas faire croître tous les différents microorganismes compris dans le produit à tester, et donc un risque de « manquer » un microorganisme. Cette étape préliminaire de préparation, rendue obligatoire par l'incapacité des algorithmes d'identification basés sur des modèles de classification à analyser de manière efficace des mélanges polymicrobiens, est donc une source d'erreur importante.

### EXPOSE DE L'INVENTION

Le but de la présente invention est de proposer un procédé d'analyse d'échantillon biologique qui permette d'analyser un échantillon biologique, indépendamment du fait que celui-ci comprennent un ou plusieurs microorganismes différents, en fonction d'une seule mesure de l'échantillon, notamment par spectroscopie, spectrométrie, ou tout type de mesure produisant un vecteur d'intensité numérique multidimensionnel.

A cet effet, l'invention a pour objet un procédé de détection dans un échantillon biologique d'au moins deux microorganismes appartenant à deux taxons différents parmi un ensemble prédéterminé $\{y_j\}$ d'un nombre de $K$ taxons de référence $y_j$ différents, chaque taxon de référence $y_j$ étant représenté par un vecteur d'intensité de référence prédéterminé $P_j$ d'un espace $R^p$, ou « prototype », obtenu en soumettant au moins un échantillon biologique de référence comprenant un microorganisme présentant le taxon de référence à une technique de mesure produisant un signal numérique multidimensionnel représentatif de l'échantillon de référence et en déterminant ledit vecteur de référence en fonction dudit signal numérique multidimensionnel, où $p$ est supérieure à 1, le procédé comportant :

- l'acquisition d'un signal numérique multidimensionnel de l'échantillon biologique par la technologie de mesure;
- la détermination d'un vecteur d'intensité $x$ de $R^p$ en fonction du signal numérique multidimensionnel acquis ;
- la construction d'un ensemble $\{\hat{\gamma}_l\}$ de modèles candidats $\hat{\gamma}_l = (\hat{\gamma}, \hat{\gamma}_0)_l$ modélisant le vecteur d'intensité $x$ selon la relation :

$$\hat{x}_l = \sum_{j=1}^{K} \hat{\gamma}_j P_j^{(a)} + \hat{\gamma}_0 I_P$$

expression dans laquelle :

- $\hat{x}_l$ est un vecteur de $R^p$ reconstruisant le vecteur d'intensité $x$ par le modèle $\hat{\gamma}_l$;
- $\hat{\gamma}_0$ est un scalaire réel et $I_p$ est le vecteur unité de $R^p$;
- $\forall j \in [[1,K]]$, $\hat{\gamma}_j$ est la j$^{ième}$ composante d'un vecteur $\hat{\gamma}$ de $R_+^K$ ;
- $\forall j \in [[1,K]]$, $P_j^{(a)} = \sum_{i=1}^{K} a_{ij} P_i$ et
- $\forall (i,j) \in [[1,K]]^2$, $a_{ij}$ est un coefficient prédéterminé;

- la sélection d'un modèle candidat $\hat{\gamma}_{sel}$ parmi l'ensemble $\{\hat{\gamma}_l\}$ des modèles candidats $\hat{\gamma}_l$, solution d'un problème selon la relation :

$$\hat{\gamma}_{sel} = \underset{\hat{\gamma}_l \in \{\hat{\gamma}_l\}}{argmin}\big(C_v(\hat{\gamma}_l) + C_c(\hat{\gamma}_l)\big)$$

expression dans laquelle :

- $C_v(\hat{\gamma}_l)$ est un critère quantifiant une erreur de reconstruction entre le vecteur d'intensité de l'échantillon biologique $x$ et la reconstruction $\hat{x}_l$ du vecteur d'intensité $x$ par un modèle candidat $\hat{\gamma}_l$ ; et
- $C_c(\hat{\gamma}_l)$ est un critère quantifiant la complexité d'un modèle candidat $\hat{\gamma}_l$;

- et la détermination de la présence dans l'échantillon biologique d'au moins deux microorganismes appartenant à des taxons différents de l'ensemble prédéterminé $\{\hat{\gamma}_l\}$ de taxons lorsqu'au moins deux composantes $\hat{\gamma}_j$ du vecteur $\hat{\gamma}$ du modèle candidat sélectionné $\hat{\gamma}_{sel}$ sont supérieures à une valeur de seuil prédéterminée strictement positive.

A cet effet l'invention a également pour objet un procédé d'identification de microorganismes présents dans un échantillon biologique parmi un ensemble prédéterminé $\{\hat{\gamma}_l\}$ d'un nombre de $K$ taxons de référence différents $y_j$, chaque taxon de référence $y_j$ étant représenté par un vecteur d'intensité de référence prédéterminé $P_j$ d'un espace $R^p$ obtenu en soumettant au moins un échantillon biologique de référence comprenant un microorganisme présentant le taxon de référence à une technique de mesure produisant un signal numérique multidimensionnel représentatif de l'échantillon de référence et en déterminant ledit vecteur de référence en fonction dudit signal numérique multidimensionnel, où $p$ est supérieure à 1, le procédé comportant :

- l'acquisition d'un signal numérique multidimensionnel de l'échantillon biologique par la technologie de mesure ;
- la détermination d'un vecteur d'intensité $x$ de $R^p$ en fonction du signal numérique multidimensionnel acquis;
- la construction d'un ensemble $\{\hat{\gamma}_l\}$ de modèles candidats $\hat{\gamma}_l = (\hat{\gamma}, \hat{\gamma}_0)_l$ modélisant le vecteur d'intensité $x$ selon la relation :

$$\hat{x}_l = \sum_{j=1}^{K} \hat{\gamma}_j P_j^{(a)} + \hat{\gamma}_0 I_P$$

expression dans laquelle :

  ○ $\hat{x}_l$ est un vecteur de $R^p$ reconstruisant le vecteur d'intensité $x$ par le modèle $\hat{\gamma}_l$;
  ○ $\hat{\gamma}_0$ est un scalaire réel et $I_P$ est le vecteur unité de $R^p$;

  ○ $\forall j \in [[1,K]]$, $\hat{\gamma}_j$ est la j$^{\text{ième}}$ composante d'un vecteur $\hat{\gamma}$ de $R_+^K$ ;

  ○ $\forall j \in [[1,K]]$, $P_j^{(a)} = \sum_{i=1}^{K} a_{ij} P_i$ ; et

  ○ $\forall (i,j) \in [[1,K]]^2$, $a_{ij}$ est un coefficient prédéterminé ;

  ■ la sélection d'un modèle candidat $\hat{\gamma}_{sel}$ parmi l'ensemble $\{\hat{\gamma}_l\}$ des modèles candidats $\hat{\gamma}_l$, solution d'un problème selon la relation :

$$\hat{\gamma}_{sel} = \underset{\hat{\gamma}_l \in \{\hat{\gamma}_l\}}{argmin}\big(C_v(\hat{\gamma}_l) + C_c(\hat{\gamma}_l)\big)$$

expression dans laquelle :

  ○ $C_v(\hat{\gamma}_l)$ est un critère quantifiant une erreur de reconstruction entre le vecteur d'intensité de l'échantillon biologique $x$ et la reconstruction $\hat{x}_l$, du vecteur d'intensité $x$ par un modèle candidat $\hat{\gamma}_l$ ; et
  ○ $C_c(\hat{\gamma}_l)$ est un critère quantifiant la complexité d'un modèle candidat $\hat{\gamma}_l$;

  ■ et la détermination de la présence dans l'échantillon biologique d'un microorganisme de taxon $y_j$ de l'ensemble prédéterminé $\{y_j\}$ pour chaque composante $\hat{\gamma}_j$ du vecteur $\hat{\gamma}$ du modèle candidat sélectionné $\hat{\gamma}_{sel}$ supérieure à une valeur de seuil prédéterminée strictement positive.

A cet effet, l'invention a également pour objet un procédé de détermination de l'abondance relative dans un échantillon biologique appartenant à deux taxons différents parmi un ensemble prédéterminé $\{y_j\}$ d'un nombre de $K$ taxons de référence $y_j$ différents, chaque taxon de référence $y_j$ étant représenté par un vecteur d'intensité de référence prédéterminé $P_j$ d'un espace $R^p$ obtenu en soumettant au moins un échantillon biologique de référence comprenant un microorganisme présentant le taxon de référence à une technique de mesure produisant un signal numérique multidimensionnel représentatif de l'échantillon de référence et en déterminant ledit vecteur de référence en fonction dudit signal numérique multidimensionnel, où $p$ est supérieure à 1, le procédé comportant :

  ■ l'acquisition d'un signal numérique multidimensionnel de l'échantillon biologique par la technologie de mesure;
  ■ la détermination d'un vecteur d'intensité $x$ de $R^p$ en fonction du signal numérique multidimensionnel acquis ;
  ■ la construction d'un ensemble $\{\hat{\gamma}_l\}$ de modèles candidats $\hat{\gamma}_l = (\hat{\gamma}, \hat{\gamma}0)_l$ modélisant le vecteur d'intensité $x$ selon la relation :

$$\hat{x}_l = \sum_{j=1}^{K} \hat{\gamma}_j P_j^{(a)} + \hat{\gamma}_0 I_P$$

expression dans laquelle :

  ○ $\hat{x}_l$ est un vecteur de $R^p$ reconstruisant le vecteur d'intensité $x$ par le modèle $\hat{\gamma}_l$;
  ○ $\hat{\gamma}_0$ est un scalaire réel et $I_p$ est le vecteur unité de $R^p$;

  ○ $\forall j \in [[1,K]]$, $\hat{\gamma}_j$ est la j$^{\text{ième}}$ composante d'un vecteur $\hat{\gamma}$ de $R_+^K$ ;

  ○ $\forall j \in [[1,K]]$, $P_j^{(a)} = \sum_{i=1}^{K} a_{ij} P_i$ ; et

∘ $\forall (i,j) \in [[1,K]]^2$, $a_{ij}$ est un coefficient prédéterminé;

■ la sélection d'un modèle candidat $\hat{\gamma}_{sel}$ parmi l'ensemble $\{\hat{\gamma}_l\}$ des modèles candidats $\hat{\gamma}_l$, solution d'un problème selon la relation :

$$\hat{\gamma}_{sel} = \underset{\hat{\gamma}_l \in \{\hat{\gamma}_l\}}{argmin}\big(C_v(\hat{\gamma}_l) + C_c(\hat{\gamma}_l)\big)$$

expression dans laquelle :

∘ $C_v(\hat{\gamma}_l)$ est un critère quantifiant une erreur de reconstruction entre le vecteur d'intensité de l'échantillon biologique $x$ et la reconstruction $\hat{x}_l$ du vecteur d'intensité $x$ par un modèle candidat $\hat{\gamma}_l$ ; et
∘ $C_c(\hat{\gamma}_l)$ est un critère quantifiant la complexité d'un modèle candidat $\hat{\gamma}_l$;

■ et la détermination de l'abondance relative dans l'échantillon biologique relative $C_j$ d'un taxon de référence $y_j$ selon la relation :

$$C \;\; = J(\hat{\gamma}_{sel})$$

expression dans laquelle $J$ est une fonction matricielle de $R_+^p \times R_+^K$ dans $R_+^K$ et $C = (C_1 \, ... \, C_j \, ... \, C_K)^T$ est un vecteur de $R_+^K$ avec $\forall j \in [[1,K]]$, $C_j$ est l'abondance relative du taxon de référence $y_j$.

Par « détection », on entend ici la détermination du caractère polymicrobien d'un échantillon biologique. L'« identification » d'un microorganisme se rapporte quant à elle à la détermination d'une donnée propre au microorganisme, par exemple son espèce, sa sous-espèce, son genre, son gram, etc... et de manière plus générale toute donnée jugée utile entrant dans la construction d'une identité unique du microorganisme.

Le terme « taxon » renvoie notamment à une notion plus large que le terme de « taxon » utilisé pour caractériser la position d'un noeud, d'une feuille ou de la racine d'une classification taxonomique du vivant. Aux termes de l'invention, le terme taxon renvoie à tout type de classification du vivant jugée utile. Notamment, l'invention s'applique à des classifications taxonomiques classiques, des classifications basées sur des phénotypes cliniques et des classifications hybrides basées sur des caractéristiques taxonomiques au sens classique du terme et des phénotypes cliniques.

Par « technique de mesure », on entend ici une mesure qui comprend la production d'un signal complexe qui est numérisé. Parmi ce type de mesure, on peut par exemple citer la spectrométrie de masse, notamment la spectrométrie MALDI-TOF et la spectrométrie ESI-MS, la spectroscopie vibrationnelle, notamment la spectroscopie RAMAN, la spectroscopie par fluorescence, notamment la spectroscopie par fluorescence intrinsèque, ou la spectroscopie infrarouge. Chacune de ces techniques produit un spectre qui est numérisé, donnant ainsi lieu à un signal numérique multidimensionnel représentatif de l'échantillon objet de la mesure.

En d'autres termes, l'invention consiste à produire des modèles candidats obtenus en mélangeant des vecteurs d'intensité représentatifs chacun d'un taxon préalablement identifié à l'aide de la technique de mesure en cause, puis à retenir le modèle candidat qui offre le meilleur compromis entre l'approximation du vecteur d'intensité de l'échantillon soumis à l'analyse et la complexité du modèle candidat. En effet, on observe que le modèle estimant le plus fidèlement l'échantillon biologique n'est pas celui qui permet la reconstruction la plus précise du vecteur d'intensité mais celui qui est à la fois suffisamment précis et de complexité modérée. Les inventeurs ont ainsi noté qu'un algorithme présentant une telle structure permet à la fois de détecter la présence de plusieurs microorganismes dans un échantillon et d'identifier les microorganismes présents dans l'échantillon avec un taux de succès élevé.

Selon un mode de réalisation de l'invention, $\forall (i,j) \in [[1,K]]^2$, $a_{ij}$ est un coefficient de similarité entre les vecteurs de référence $P_i$ et $P_j$ des taxons de référence $y_i$ et $y_j$. Notamment, les coefficients de similarité peuvent être définis comme les produits scalaires entre les vecteurs d'intensité, normalisés ou non, ou, lorsque les vecteurs de référence listent des pics compris dans des spectres produits par la technique de mesure, comme leurs coefficients de Jaccard. On observe en effet que la proximité biologique entre deux taxons différents induit une proximité entre les deux vecteurs de référence de ces taxons. Un microorganisme de taxon de référence $y_j$ peut ainsi être identifié dans un échantillon biologique en supplément de, ou en lieu et place de, d'un microorganisme de taxon de référence $y_i$ dont le vecteur de

référence $P_i$ est très proche du vecteur de référence $P_j$ du taxon $y_j$. La création de vecteurs de référence ajustés $P_j^{(a)} =$ $\sum_{i=1}^{K} a_{ij} P_i$ tenant compte de la proximité biologique entre des taxons de référence minimise donc les erreurs de détection et d'identification.

Selon un mode de réalisation, $\hat{\gamma}_0 = 0$, et la construction de l'ensemble $\{\hat{\gamma}_l\}$ des modèles candidats $\hat{\gamma}_l = (\hat{\gamma},0)_l$ comporte la résolution d'un ensemble de problèmes d'optimisation pour des valeurs d'un paramètre $\lambda$ de $R_+$, chaque problème étant défini selon la relation :

$$\hat{\gamma}(\lambda) = \underset{\gamma \in R_+^K}{\operatorname{argmin}} \left( \left\| x - \sum_{j=1}^{K} \gamma_j P_j^{(a)} \right\|^2 + \lambda |\gamma|_1 \right)$$

expression dans laquelle $|\gamma|_1$ est la norme L1 du vecteur $\gamma$. En d'autres termes, la construction des modèles candidats comprend une pénalité de type LASSO faisant intervenir un premier terme comportant l'erreur de construction $x - \sum_{j=1}^{K} \gamma_j P_j^{(a)}$ et un second terme de pondération $|\gamma|_1$ à base de norme L1. Pour un terme $\lambda$ nul, le modèle candidat obtenu est celui qui minimise l'erreur de reconstruction sous une contrainte de positivité des coefficients du modèle. Comme dit plus haut, ce modèle n'est généralement pas celui qui estime le mieux l'échantillon biologique car il présente usuellement la complexité la plus élevée en raison des composantes $\hat{\gamma}_j$ en majorité, voire en totalité, non nulles. A mesure que le paramètre $\lambda$ augmente, on observe que les composantes $\hat{\gamma}_j$ deviennent nulles une part une et les unes après les autres. En parcourant les valeurs de $\lambda$, on obtient donc un ensemble de modèles candidats ayant chacun une structure de $\hat{\gamma}$ unique. L'application de ce type d'algorithme permet donc de réaliser une présélection d'un nombre réduit de structures de modèles parmi les $2^K$ structures de modèle possibles. Comme par ailleurs, chaque problème d'optimisation est convexe, il est possible de calculer très rapidement les modèles candidats. Une accélération sensible du procédé selon l'invention est ainsi obtenue.

En variante, le scalaire $\gamma_0$ est non nul, et le problème d'optimisation décrit ci-dessus se réécrit selon la relation :

$$(\hat{\gamma}(\lambda), \hat{\gamma}_0(\lambda)) = \underset{\gamma \in R_+^K, \gamma_0 \in R_+}{\operatorname{argmin}} \left( \left\| x - \left( \sum_{j=1}^{K} \gamma_j P_j^{(a)} + \gamma_0 I_P \right) \right\|^2 + \lambda |\gamma|_1 \right)$$

Des structures différentes peuvent ainsi être sélectionnées.

Selon un autre mode de réalisation, $\gamma_0 = 0$, et la construction de $\{\hat{\gamma}_l\}$ des modèles candidats $\hat{\gamma}_l = (\hat{\gamma},0)_l$ comporte la résolution d'un ensemble de problèmes d'optimisation pour des valeurs de paramètres $\lambda$ et $\beta$ de $R_+$, chaque problème étant défini selon la relation :

$$\hat{\gamma}(\lambda, \beta) = \underset{\gamma \in R_+^K}{\operatorname{argmin}} \left( \left\| x - \sum_{j=1}^{K} \gamma_j P_j^{(a)} \right\|^2 + \lambda |w_1 \odot \gamma|_1 + \beta |w_2 \odot \gamma|_2 \right)$$

expression dans laquelle :

- $| |_1$ est la norme L1;
- $| |_2$ est la norme L2;
- $a \odot b$ est le produit terme à terme des vecteurs a et b ; et
- $w_1$ et $w_2$ sont des vecteurs de poids prédéterminés de $R_+^K$.

En d'autres termes, la construction des modèles candidats par la méthode LASSO est mise en oeuvre à l'aide d'un algorithme de type LARS-EN (pour « Least Angle Regression - Elastic Net) avec pénalisation de type « *elastic net* » ($\beta = 0$) combiné avec une pénalité adaptative ($w_1 = w_2 = I_K$). L'algorithme LARS-EN est par exemple celui de Zou et Hastie qui est compris dans le module « *R elasticNet* » disponible à l'adresse http://cran.r-project.org/web/packages/elasticnet/. En variante, seul la pénalité de type « *elastic net* » est mise en oeuvre, c'est-à-dire que $\beta$ est posé nul, ou seul la pénalité de type LASSO adaptatif est mise en oeuvre, c'est-à-dire que $w_1$ et $w_2$ sont posés égaux au vecteur unité $I_K$ de $R^K$. Des structures différentes pour les modèles candidats peuvent être obtenues. De manière avantageuse, la version adaptative permet d'inclure de l'information a priori sur les taxons qui sont susceptibles d'être contenus dans l'échantillon biologique. Par exemple, en choisissant une composante des vecteurs $w_1$ $w_2$ plus faible que les autres composantes, permet de rendre plus vraisemblable la présence du taxon correspondant à cette composante dans l'échantillon biologique.

En variante, le scalaire $\hat{\gamma}_0$ est non nul, et le problème d'optimisation décrit ci-dessus se réécrit selon la relation :

$$(\hat{\gamma}(\lambda,\beta), \hat{\gamma}_0(\lambda,\beta)) = \underset{\gamma \in R_+^K, \gamma_0 \in R_+}{\text{argmin}} \left( \left\| x - \left( \sum_{j=1}^{K} \gamma_j P_j^{(a)} + \gamma_0 I_P \right) \right\|^2 + \lambda |w_1 \odot \gamma|_1 + \beta |w_2 \odot \gamma|_2 \right)$$

D'autres approches permettant une présélection autre qu'un algorithme de type LARS-EN peuvent être envisagées, comme par exemple un algorithme de type « *stepwise* » simple ou structuré, comme par exemple décrit dans le document « Structured, sparse regression with application to HIV drug resistance » de Daniel Percival et al., Annals of Applied Statistics, 2011, vol.5, No.2A, 628-644, voire une approche exhaustive visant à évaluer un nombre important de structures de modèle candidat parmi les $2^K$ structures possibles.

De manière avantageuse, pour chaque vecteur $\hat{\gamma}$ solution d'un problème d'optimisation, un nouveau modèle candidat $\hat{\gamma}_l = (\hat{\gamma}^{lm}, \hat{\gamma}_0^{lm})_l$ est calculé, et remplace le modèle $\hat{\gamma}_l = (\hat{\gamma}, 0)_l$ correspondant au vecteur $\hat{\gamma}$, les composantes du vecteur $\hat{\gamma}^{lm}$ du nouveau modèle $\hat{\gamma}_l = (\hat{\gamma}^{lm}, \hat{\gamma}_0^{lm})_l$, correspondant aux composantes nulles du vecteur $\hat{\gamma}$, étant forcées à zéro, et le nouveau modèle $\hat{\gamma}_l = (\hat{\gamma}^{lm}, \hat{\gamma}_0^{lm})_l$ étant calculé en résolvant le problème d'optimisation selon les relations:

$$(\hat{\gamma}^{lm}, \hat{\gamma}_0^{lm}) = \underset{\substack{\gamma_0^{lm} \in R_+ \\ \gamma^{lm} \in R_+^K}}{\text{argmax}} \left( -\frac{p}{2} ln(2\pi\sigma(x_l)^2) - \frac{1}{2\sigma(x_l)^2} \sum_{b=1}^{p} (x_b - x_{lb})^2 \right)$$

$$\sigma(x_l)^2 = \frac{1}{p} \sum_{b=1}^{p} (x_b - x_{lb})^2$$

$$x_l = \gamma_0^{lm} I_p + \sum_{j:\hat{\gamma}_j \neq 0} \gamma_j^{lm} P_j^{(a)}$$

expression dans laquelle :

- $x_b$ est la b$^{\text{ième}}$ composante du vecteur d'intensité de l'échantillon biologique $x$ ; et

- $x_{lb}$ est la b$^{\text{ième}}$ composante du vecteur de reconstruction $x_l = \gamma_0^{lm} I_p + \sum_{j:\hat{\gamma}_j > 0} \gamma_j^{lm} P_j^{(a)}$.

En d'autres termes, les modèles candidats sont recalculés par un modèle linéaire standard en conservant les structures de vecteurs obtenues à l'issue de la mise en oeuvre de l'approche LASSO ou analogue. En raison de la pondération de l'erreur de reconstruction par un terme à base de norme L1, les modèles candidats obtenus par une approche LASSO présentent une vraisemblance réduite, bien qu'ils présentent des structures pertinentes. Les modèles candidats sont avantageusement recalculés en conservant les structures déterminées par l'approche LASSO et en maximisant leur vraisemblance telle que définie dans un modèle linéaire standard. La qualité de l'analyse de l'échantillon biologique est ainsi renforcée puisque la sélection des modèles candidats et l'estimation de leur effet sont réalisées en deux étapes distinctes.

Selon un mode de réalisation, le critère $C_v(\hat{\gamma}_l)$ quantifiant l'erreur de reconstruction est un critère de vraisemblance. Plus particulièrement :

$$C_v(\hat{\gamma}_l) = -\frac{p}{2} ln(2\pi\hat{\sigma}^2) - \frac{1}{2\hat{\sigma}^2} \sum_{b=1}^{p} (x_b - \hat{x}_{lb})^2$$

expression dans laquelle :

- $\hat{\sigma}^2 = \frac{1}{p} \sum_{b=1}^{p} (x_b - \hat{x}_{lb})^2$ ;
- $x_b$ est la b$^{ième}$ composante du vecteur d'intensité de l'échantillon biologique $x$ ; et
- $\hat{x}_{lb}$ est la b$^{ième}$ composante du vecteur de reconstruction $\hat{x}_l$ du modèle candidat $\hat{\gamma}_l$.

Selon un mode de réalisation, le critère $C_c(\hat{\gamma}_l)$ quantifiant la complexité du modèle $\hat{\gamma}_l$ quantifie ladite complexité en termes de nombre de composantes $\hat{\gamma}_j$ du vecteur $\gamma$ strictement positives. Plus particulièrement :

$$\text{Si } \hat{\gamma}_0 = 0 \text{ alors} \qquad C_c(\hat{\gamma}_l) = \left( 1 + \sum_{j=1}^{K} \mathbf{1}\left(\hat{\gamma}_j > 0\right) \right) \ln p$$

$$\text{Si } \hat{\gamma}_0 \neq 0 \text{ alors} \qquad C_c(\hat{\gamma}_l) = \left( 2 + \sum_{j=1}^{K} \mathbf{1}\left(\hat{\gamma}_j > 0\right) \right) \ln p$$

expression dans laquelle la fonction $\mathbf{1}(.)$ est égale à 1 si son argument est vrai et zéro sinon.

En variante, lorsque le scalaire $\hat{\gamma}_0$ est posé égal à zéro lors du calcul des modèles candidats, et donc le scalaire $\gamma_0^{lm}$, le critère $C_c(\hat{x}_l)$ se réécrit selon la relation :

$$C_c(\hat{\gamma}_l) = \left( 1 + \sum_{j=1}^{K} \mathbf{1}\left(\hat{\gamma}_j > 0\right) \right) \ln p$$

Ainsi, selon un mode de réalisation privilégié, le modèle candidat $\hat{\gamma}_{sel}$ sélectionné est celui qui minimise la fonction selon la relation :

$$-\frac{p}{2} ln(2\pi\hat{\sigma}^2) - \frac{1}{2\hat{\sigma}^2} \sum_{b=1}^{p} (x_b - \hat{x}_{lb})^2 + \left( 2 + \sum_{j=1}^{K} \mathbf{1}\left(\hat{\gamma}_j > 0\right) \right) ln p$$

ou la fonction selon la relation :

$$-\frac{p}{2}ln(2\pi\hat{\sigma}^2) - \frac{1}{2\hat{\sigma}^2}\sum_{b=1}^{p}(x_b - \hat{x}_{lb})^2 + \left(1 + \sum_{j=1}^{K}\mathbf{1}\left(\hat{\gamma}_j > 0\right)\right)ln\,p$$

En d'autres termes, le modèle candidat sélectionné est celui qui minimise un critère « BIC » (acronyme de « *Bayesian Information Criterion* ») qui propose une sélection performante du modèle. On pourra par exemple se reporter au document « Le critère BIC : fondements théoriques et interprétation », d'Emilie Labarbier et Tristant Mary-Huard, INRIA, Rapport de recherche n°5315, Septembre 2004, pour une description plus détaillée de ce critère.

D'autres critères de sélection sont cependant possibles, comme par exemple un critère « AIC » (acronyme de « *Akaike Information Criterion* »), « MLD » (acronyme de « *Minimum Rescription Length* »), « Cp de Mallows », ou de manière générale tout critère combinant un critère de vraisemblance ou d'erreur de reconstruction avec un critère de complexité.

Selon un mode de réalisation, les taxons appartiennent à un même niveau taxonomique, notamment le niveau espèce, genre ou sous-espèce. En variante, les taxons appartiennent à au moins deux niveaux taxonomiques différents, notamment des espèces, des genres, et/ou des sous-espèces. Notamment, si un degré de similarité entre un ensemble de taxons définis au sein d'un premier niveau taxonomique est supérieur à un seuil prédéterminé, alors pour la constitution de l'ensemble prédermiíné $\{y_j\}$ des taxons de référence, lesdits taxons sont regroupés et remplacés par un taxon de référence défini à un deuxième niveau taxonomique, supérieur au premier niveau taxonomique.

En d'autres termes, le procédé selon l'invention est libre de choisir des niveaux de description différents des microorganismes. Par exemple, il est possible de combiner des espèces avec des genres sans que cela ne pose de problème particulier. Grâce à l'invention, il est donc possible de choisir des taxons de référence qui se distinguent suffisamment les uns des autres au regard des vecteurs de référence, et ainsi minimiser les erreurs de détection et d'identification. Par exemple, lorsque les spectres d'espèces au sein d'un même genre particulier présentent des degrés de similarité très importants, risquant de ce fait de mettre l'algorithme de détection ou d'identification en difficulté, il est possible de choisir plutôt le genre que les espèces, tout en continuant de privilégier le niveau espèce pour les autres microorganismes.

Selon un mode de réalisation, des taxons appartiennent à un premier niveau taxonomique, et un nouveau modèle du vecteur *x* est calculé en estimant la contribution desdits taxons à un deuxième niveau taxonomique, supérieur au premier niveau taxonomique, en sommant les composantes du vecteur $\hat{\gamma}$ rattachées audit niveau supérieur. Notamment, le modèle du vecteur *x* est calculé pour le niveau taxonomique supérieur si un degré de similarité au sein du premier niveau est supérieur à un seuil prédéterminé.

En d'autres termes, grâce à l'invention, il est possible d'identifier le niveau taxonomique supérieur d'un microorganisme grâce aux résultats obtenus par l'algorithme au niveau taxonomique inférieur. Ceci permet par exemple de conserver un niveau taxonomique identique pour tous les microorganismes, quand bien même des microorganismes présenteraient une similarité très élevée audit niveau, et de compenser les erreurs de détection et d'identification qui en découlent en calculant un modèle candidat pour le niveau taxonomique supérieur. Cette approche est également applicable à des taxons de référence considérés à des niveaux différents, des niveaux supérieurs pouvant à la demande être calculés, notamment lorsque le modèle candidat finalement sélectionné comporte des taxons jugés très similaires.

Selon un mode de réalisation, la technique de mesure produit un spectre et les vecteurs d'intensité de référence $P_j$ sont des listes de pics compris dans les spectres des taxons de référence $y_j$. Notamment, la technique de mesure comprend une spectrométrie de masse.

Selon un mode de réalisation :

$$C_j = \frac{\hat{\gamma}_{j,sel}}{\sum_{i=1}^{K}\hat{\gamma}_{i,sel}}$$

expression dans laquelle $\forall j \in [[1,K]]$, $\hat{\gamma}_{j,sel}$ est la j$^{ième}$ composante du vecteur $\hat{\gamma}$ du modèle sélectionné $\hat{\gamma}_{sel}$.

L'invention a également pour objet un dispositif d'analyse d'un échantillon biologique comprenant :

- un spectromètre ou un spectroscope apte à produire des spectres de l'échantillon biologique ;
- une unité de calcul apte à mettre en oeuvre un procédé du type précité.

**BREVE DESCRIPTION DES FIGURES**

L'invention sera mieux comprise à la lecture de la description qui va suivre, donnée uniquement à titre d'exemple, et faite en relation avec les dessins annexés, dans lesquels :

- la figure 1 est un organigramme illustrant un procédé selon l'invention ;
- les figures 2A et 2B sont respectivement une matrice de similarité entre plusieurs espèces de bactéries utilisées pour tester le procédé selon l'invention et des vecteurs de pics de mélanges desdites bactéries ; et
- les figures 3A et 3B sont respectivement des résultats de la détection et de l'identification selon l'invention respectivement réalisées au niveau espèce et au niveau genre sur les mélanges.

## DESCRIPTION DETAILLEE DE L'INVENTION

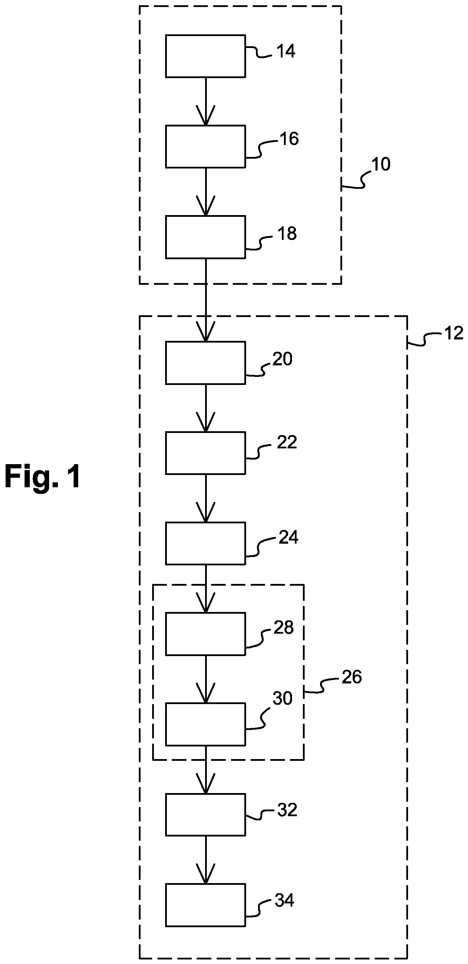

Il va à présent être décrit en relation avec l'organigramme de la figure 1 un mode de réalisation de l'invention appliquée à la spectrométrie de masse MALDI-TOF (acronyme de « _Matrix-assisted lasser desorption/ionization time of flight_ ») et pour un unique niveau taxonomique, à savoir le niveau espèce. La spectrométrie de masse MALDI-TOF est bien connue en soi et ne sera donc pas décrite plus en détail par la suite. On pourra, par exemple, se référer au document de Jackson O. Lay, « Maldi-tof spectrometry of bacteria », Mass Spectrometry Reviews, 2001, 20, 172-194.

Le procédé débute par une étape **10** de construction d'un ensemble $\{P_j\} = \{P_1 \, P_2 \, ... \, P_K\}$ de $K$ vecteurs d'intensité de référence $P_j$, chacun associé à une espèce de référence $y_j$ de microorganisme préalablement identifié, et se poursuit par une étape **12** d'analyse d'un échantillon biologique dont on cherche à savoir s'il comporte une ou plusieurs espèces de référence différentes et/ou dont on cherche à identifier la ou les espèces de référence qu'il est susceptible de contenir et/ou dont on cherche à quantifier l'abondance des microorganismes présents dans l'échantillon.

Un exemple de réalisation de l'étape **10** est à présente décrit pour une espèce de référence $y_j$. L'étape **10** comprend l'acquisition, en **14,** d'au moins un spectre de masse numérique de l'espèce $y_j$ dans une gamme de Thomson $[m_{min} ; m_{max}]$ prédéterminée par une spectrométrie MALDI-TOF. Par exemple, plusieurs souches d'un microorganisme appartenant à l'espèce $y_j$ sont utilisées et un spectre est acquis pour chacune des souches. Les spectres numériques acquis pour l'espèce $y_j$ sont ensuite prétraités, avantageusement après une transformation logarithmique, afin notamment de débruiter ceux-ci et ôter leur ligne de base, d'une manière connue en soi.

Une identification des pics présents dans les spectres acquis est alors réalisée en **16,** par exemple au moyen d'un algorithme de détection de pics basé sur la détection de maxima locaux. Une liste des pics pour chaque spectre acquis, comportant la localisation et l'intensité des pics du spectre, est ainsi produite.

Le procédé se poursuit, en **18,** par une étape de quantification, ou « _binning_ ». Pour ce faire la gamme des Thompsons $[m_{min} ; m_{max}]$ est subdivisée en $p$ intervalles, ou « _bins_ », de largueurs prédéterminées, par exemple identiques. Chaque liste de pics est réduite en ne retenant qu'un seul pic par intervalle, par exemple le pic présentant la plus forte intensité. Chaque liste est ainsi réduite à un vecteur de $R^p$ ayant pour composant l'intensité des pics retenus pour les intervalles de quantification, la valeur nulle pour une composante signifiant qu'aucun pic n'a été détecté, et donc conservé, dans l'intervalle correspondant. Un vecteur numérique multidimensionnel $P_j \in R^p$, également appelé « prototype », est ensuite produit pour l'espèce $y_j$ en fonction des listes de pics réduites. Chaque composante du vecteur $P_j$ est notamment posée nulle si la fréquence des composantes correspondantes des listes réduites qui sont strictement positives est inférieure à un seuil, par exemple 30%, et sinon est choisie égale à la valeur médiane des composantes correspondantes des listes réduites qui sont strictement positives ou égales à la moyenne des composantes correspondantes des listes réduites.

Notamment, pour la spectrométrie MALDI-TOF, $[m_{min} ; m_{max}]$ [3000;17000]. En effet, il a été observé que les informations suffisantes à l'identification des micro-organismes sont regroupées dans cette gamme de rapport masse sur charge, et qu'il n'est donc pas besoin de tenir compte d'une gamme plus large. La gamme $[m_{min} ; m_{max}]$ est subdivisée en $p$ = 1300 intervalles constants. Le vecteur $P_j$ est donc un vecteur de $R^{1300}$. En variante, la largeur des intervalles est croissante de façon logarithmique, comme décrit dans la demande EP 2 600 385.

En variante, le vecteur $P_j$ est « binarisé » en posant la valeur d'une composante du vecteur $P_j$ à « 1 » lorsqu'un pic est présent dans l'intervalle correspondant, et à « 0 » lorsqu'aucun pic n'est présent pas dans cet intervalle. Ceci a pour effet de rendre plus robuste l'analyse d'échantillon biologique de l'étape **12**. Les inventeurs ont en effet noté que l'information pertinente notamment pour l'identification d'une bactérie est contenue essentiellement dans l'absence et/ou la présence de pics, et que l'information d'intensité est moins pertinente. En outre, on observe que l'intensité est une grandeur très variable d'un spectre à l'autre et/ou d'un spectromètre à un autre. Du fait de cette variabilité, il est difficile de prendre en compte les valeurs brutes d'intensité dans les outils de classification.

Bien entendu, le vecteur $P_j$ de l'espèce $y_j$ peut être obtenu par toute manière jugée utile afin de produire un vecteur représentatif de l'espèce $y_j$. Par exemple, les spectres des souches de l'espèce $y_j$ font l'objet d'un traitement statistique afin de produire un unique spectre. Le spectre unique fait ensuite l'objet d'une détection de pics et la liste de pics produite est alors quantifiée en conservant dans chaque intervalle de la quantification que le pic de plus forte intensité. Le traitement statistique peut par exemple être le calcul de la moyenne des spectres, le calcul d'un spectre médian, ou la sélection du spectre qui présente la distance moyenne à tous les autres spectres de l'espèce la plus faible. De même, l'étape de quantification **18,** qui permet de réduire de manière importante le nombre de données à traiter tout en garantissant une robustesse algorithmique, est optionnelle. Le vecteur $P_j$ peut par exemple être constitué du spectre numérique directement obtenu après l'étape **14** d'acquisition et de prétraitement. D'une manière générale, toute méthode permettant de produire pour l'espèce $y_j$ un vecteur numérique comportant une signature unique de cette espèce peut convenir.

Les vecteurs $\{P_j\}$ obtenus par l'étape **10** de construction sont alors mémorisés dans une base de données. La base de données est ensuite incorporée dans un système d'analyse d'échantillons biologiques par spectrométrie de masse comprenant un spectromètre de masse, de type MALDI-TOF, ainsi qu'une unité de traitement d'informations, connectée au spectromètre et apte à recevoir, numériser et traiter les spectres de masse acquis en mettant en oeuvre l'étape d'analyse **12.** Le système d'analyse peut également comporter une unité de traitement d'informations distante du spectromètre de masse. Par exemple, l'analyse numérique est réalisée sur un serveur distant accessible par un utilisateur au moyen d'un ordinateur personnel connecté au réseau internet auquel est également connecté le serveur. L'utilisateur charge des spectres de masse numérique non traités obtenus par un spectromètre de masse de type MALDI-TOF sur le serveur, et ce dernier met alors en oeuvre l'algorithme d'analyse et renvoie les résultats de l'algorithme à l'ordinateur de l'utilisateur. On notera que la base de données, notamment celle embarquée dans le système d'analyse, peut être mise à jour à tout moment, notamment pour ajouter, remplacer et/ou retirer un vecteur d'intensité de référence.

Il va à présent être décrit un exemple de réalisation de l'étape d'analyse **12** d'un échantillon biologique dont on cherche à savoir s'il comprend un ou plusieurs types de microorganisme et/ou dont on cherche à identifier le ou les microorganismes qu'il contient et/ou dont on cherche à quantifier l'abondance relative de plusieurs microorganismes présents dans l'échantillon.

L'étape d'analyse **12** comprend une première étape **20** de préparation de l'échantillon biologique à la spectrométrie MALDI-TOF, notamment l'incorporation de l'échantillon dans une matrice, comme cela est connu en soi. Plus particulièrement, l'échantillon ne subit aucune étape préliminaire visant à isoler les différents types de microorganismes qu'il contient.

L'analyse **12** se poursuit par une étape **22** d'acquisition d'un spectre de masse numérique de l'échantillon biologique par un spectromètre MALDI-TOF et le spectre acquis est débruité et sa ligne de base retirée.

Dans une étape **24** suivante, une détection des pics du spectre numérique et de détermination d'un vecteur d'intensité $x$ de $R^p$ à partir des pics détectés. Par exemple, une quantification de l'espace de Thomson telle que décrite précédemment est mise en oeuvre en ne conservant que le pic de plus forte intensité dans un intervalle de quantification. D'une manière générale, le vecteur d'intensité $x$ peut être généré par toute méthode appropriée.

Une fois le vecteur d'intensité $x$ de $R^p$ obtenu en fonction du spectre de masse de l'échantillon biologique, l'analyse se poursuit, en **26,** par la construction d'un ensemble $\{\hat{\gamma}_l\}$ de modèles candidats $\hat{\gamma}_l = (\hat{\gamma}_j, \hat{\gamma}_0)_l$ modélisant le vecteur d'intensité $x$ selon la relation :

$$\hat{x}_l = \sum_{j=1}^{K} \hat{\gamma}_j P_j^{(a)} + \hat{\gamma}_0 I_P \qquad (1)$$

expression dans laquelle :

- $\hat{x}_l$ est un vecteur de $R^p$ reconstruisant le vecteur d'intensité $x$ par le modèle $\hat{\gamma}_l$;
- $K$ est le nombre de vecteurs d'intensité de référence $P_j$ mémorisés dans la base de données;
- $\hat{\gamma}_0$ est un scalaire réel et $I_P = (11 \ldots 1)^T$ est le vecteur unité de $R^p$;
- $\forall j \in [[1,K]]$, $\hat{\gamma}_j$ est la $j^{\text{ième}}$ composante d'un vecteur $\hat{\gamma}$ de $R_+^K$ ;
- $\forall j \in [[1,K]]$, $P_j^{(a)} = \sum_{i=1}^{K} a_{ij} P_i$ ; et
- $\forall(i,j) \in [[1,K]]^2$, $a_{ij}$ est un coefficient prédéterminé ;

Plus particulièrement, les coefficients $a_{ij}$ sont des coefficients quantifiant la similarité, ou la « proximité » entre les vecteurs d'intensité de référence $P_j$, notamment des coefficients de Jaccard selon la relation :

$$a_{ij} = \frac{N_{ij}^C}{(N_i + N_j) - N_{ij}^C} \qquad (2)$$

où $N_i$ est le nombre de composantes non nulles du vecteur $P_i$, $N_j$ est le nombre de composantes non nulles du vecteur $P_j$, et $N_{ij}^C$ est le nombre de composantes non nulles que partagent les vecteurs $P_i$ et $P_j$.

Plus particulièrement, la construction **26** de l'ensemble $\{\hat{\gamma}_l\}$ comporte une première étape **28** de sélection d'un ensemble $\{\tilde{\gamma}\}$ de structures $\tilde{\gamma}$ de complexité croissantes pour les vecteurs $\hat{\gamma}$ des modèles candidats $\hat{\gamma}_l$, suivie d'une étape **30** de calcul de modèles candidats ayant les structures de $\tilde{\gamma}$ sélectionnées.

Notamment, l'étape **28** consiste à sélectionner un ensemble $\{\tilde{\gamma}\}$ de vecteurs binaires $\tilde{\gamma}$ de $R^K$ comprenant un nombre croissants de composantes nulles, chaque vecteurs $\tilde{\gamma}$ indiquant quelles composantes du vecteurs $\hat{\gamma}$ d'un modèle candidat $\hat{x}_l$ sont libres ou forcées à 0. Notamment, une composante de valeur 0 du vecteur $\tilde{\gamma}$ indique que la composante correspondante du vecteur $\hat{\gamma}$ est forcée à 0, et une composante de valeur 1 du vecteur du vecteur $\tilde{\gamma}$ indique que la composante correspondante du vecteur $\hat{\gamma}$ est libre de prendre une valeur positive non nulle. Par exemple, en posant $p$ = 3, et en choisissant un vecteur $\tilde{\gamma} = (1\ 0\ 1)^T$, il est déterminé qu'un modèle candidat $\hat{x}_l$ sera calculé en ayant la deuxième composante du vecteur $\hat{\gamma}$ forcée à zéro et les première et troisième composantes du vecteur $\hat{\gamma}$ libres de prendre des valeurs positives non nulles.

De manière avantageuse, les structures $\tilde{\gamma}$ des vecteurs $\hat{\gamma}$ sont sélectionnées en mettant en oeuvre une approche, ou « pénalisation », LASSO, c'est-à-dire en résolvant un ensemble de problèmes d'optimisation pour des valeurs d'un paramètre $\lambda$ de $R_+$, chaque problème étant défini selon la relation :

$$(\hat{\gamma}(\lambda), \hat{\gamma}_0(\lambda)) = \operatorname*{argmin}_{\gamma \in R_+^K, \gamma_0 \in R_+} \left( \left\| x - \left( \sum_{j=1}^{K} \gamma_j P_j^{(a)} + \gamma_0 I_P \right) \right\|^2 + \lambda |\gamma|_1 \right) \qquad (3)$$

expression dans laquelle $|\gamma|_1$ est la norme L1 du vecteur $\gamma$.

Notamment en partant de $\lambda = 0$, correspondant à un vecteur $\hat{\gamma}(0)$ dont chaque composante est libre de prendre n'importe quelle valeurs positives ou nulle, à mesure que le paramètre $\lambda$ croit, la pénalisation LASSO annule une part une des composantes du vecteur $\hat{\gamma}(\lambda)$ jusqu'à aboutir à un vecteur $\hat{\gamma}(\lambda)$ nul. Il est ainsi obtenu un nombre réduit, c'est-à-dire très inférieur à $2^K$, le plus souvent proche ou égal à $K$, vecteurs $\hat{\gamma}(\lambda)$ de structures différentes $\tilde{\gamma}(\lambda)$. Par ailleurs, l'approche LASSO visant à minimiser l'erreur de reconstruction $\left\| x - \left( \sum_{j=1}^{K} \gamma_j P_j^{(a)} + \gamma_0 I_P \right) \right\|$ sous contrainte, chacune des structures sélectionnées représente une structure pertinente, voire la meilleure structure, pour la complexité qu'elle présente, c'est-à-dire le nombre de ses composantes nulles. L'approche LASSO, et ses variantes comme la pénalité « elastic net », est par exemple mis en oeuvre à l'aide de l'algorithme LARS-EN de Zou et Hastie qui est compris dans le module « R elasticNet » disponible à l'adresse http://cran.r-project.org/web/packages/elasticnet/.

Pour chaque structure $\tilde{\gamma}$ sélectionnée, l'étape **30** de calcul du modèle candidat $\hat{\gamma}_l$ ayant un vecteur $\hat{\gamma}$ selon la structure $\tilde{\gamma}$ consiste de manière privilégiée à maximiser un critère de vraisemblance entre le vecteur de reconstruction $\hat{x}_l$ du modèle $\hat{\gamma}_l$ et le vecteur d'intensité $x$ de l'échantillon biologique. Notamment, le modèle candidat $\hat{\gamma}_l = (\hat{\gamma}, \hat{\gamma}_0)$ est calculé en résolvant le problème d'optimisation selon les relations :

$$(\hat{\gamma}, \hat{\gamma}_0) = \operatorname*{argmax}_{\substack{\gamma_0 \in R_+ \\ \gamma \in R_+^K}} \left( -\frac{p}{2} \ln(2\pi\sigma(x_l)^2) - \frac{1}{2\sigma(x_l)^2} \sum_{b=1}^{p} (x_b - x_{lb})^2 \right) \qquad (4)$$

$$\sigma(x_l)^2 = \frac{1}{p} \sum_{b=1}^{p} (x_b - x_{lb})^2 \qquad (5)$$

$$x_l = \gamma_0 I_p + \sum_{j=1}^{K} (\tilde{\gamma} \odot \gamma)_j \, P_j^{(a)} \qquad (6)$$

expression dans laquelle :

- $a \odot b$ est le produit terme à terme des vecteurs a et b ;
- $x_b$ est la b$^{ième}$ composante du vecteur d'intensité de l'échantillon biologique $x$ ; et
- $x_{lb}$ est la b$^{ième}$ composante du vecteur de reconstruction $x_l$.

De manière équivalente lorsque les structures $\tilde{\gamma}$ sont déterminées par l'approche LASSO de la relation (3), le modèle candidat $\hat{\gamma}_l = \left(\hat{\gamma}^{lm}, \hat{\gamma}_0^{lm}\right)_l$ a les composantes de son vecteur $\hat{\gamma} = \hat{\gamma}^{lm}$ qui sont forcées à 0 lorsque les composantes correspondantes du vecteur $\tilde{\gamma}$ sont égales à 0, ce qui correspond à un vecteur de reconstruction $\hat{x}_l$ qui se réécrit $\hat{x}_l = \hat{\gamma}_0^{lm} I_p + \sum_{j:\hat{\gamma}_j > 0} \hat{\gamma}_j^{lm} P_j^{(a)}$. Le problème d'optimisation des relations (4), (5), (6) se réécrit alors selon les relations :

$$\left(\hat{\gamma}^{lm}, \hat{\gamma}_0^{lm}\right) = \underset{\substack{\gamma_0^{lm} \in R_+ \\ \gamma^{lm} \in R_+^K}}{argmax} \left(-\frac{p}{2} ln(2\pi\sigma(x_l)^2) - \frac{1}{2\sigma(x_l)^2} \sum_{b=1}^{p} (x_b - x_{lb})^2\right) \qquad (4bis)$$

$$\sigma(x_l)^2 = \frac{1}{p} \sum_{b=1}^{p} (x_b - x_{lb})^2 \qquad (5)$$

$$x_l = \gamma_0^{lm} I_p + \sum_{j:\hat{\gamma}_j \neq 0} \gamma_j^{lm} P_j^{(a)} \qquad (6bis)$$

expression dans laquelle $j:\hat{\gamma}_j \neq 0$ signifie les composantes $j$ du vecteur $\hat{\gamma}$ calculé par l'approche LASSO qui sont non nulles, donc strictement positives.

Une fois l'ensemble $\{\hat{\gamma}_l\}$ des modèles candidats $\hat{\gamma}_l$ calculé, l'étape **12** d'analyse de l'échantillon biologique se poursuit par une étape **32** de sélection d'un modèle candidat $\hat{\gamma}_{sel} = (\hat{\gamma}_{sel}, \hat{\gamma}_{0sel})$ parmi l'ensemble $\{\hat{\gamma}_l\}$, le modèle candidat sélectionné $\hat{\gamma}_{sel}$ étant celui considéré comme estimant de manière la plus pertinente le vecteur d'intensité $x$ de l'échantillon biologique analysé.

Plus particulièrement, la sélection du modèle candidat $\hat{\gamma}_{sel}$ consiste à choisir le modèle qui offre le meilleur compromis entre l'approximation du vecteur $x$ et la complexité de la structure du modèle. Pour ce faire, le modèle $\hat{\gamma}_{sel}$ est celui qui minimise un critère mélangeant un critère $C_v(\hat{\gamma})$ quantifiant l'erreur de reconstruction de l'estimation, ou reconstruction, du vecteur $x$ et un critère $C_c(\hat{\gamma})$ quantifiant la complexité de l'estimation, et notamment le nombre de composantes du vecteur $\hat{\gamma}$ non nulles. Avantageusement, le modèle $\hat{\gamma}_{sel}$ est sélectionné en minimisant un critère « BIC », le modèle $\hat{\gamma}_{sel}$ étant la solution du problème d'optimisation selon la relation :

$$\hat{\gamma}_{sel} = \underset{\hat{\gamma}_l \in \{\hat{\gamma}_l\}}{argmin} \left(C_v(\hat{\gamma}_l) + C_c(\hat{\gamma}_l)\right) \qquad (7)$$

$$C_v(\hat{\gamma}_l) = -\frac{p}{2} ln(2\pi\hat{\sigma}^2) - \frac{1}{2\hat{\sigma}^2} \sum_{b=1}^{p} (x_b - \hat{x}_{lb})^2 \qquad (8)$$

$$C_c(\hat{\gamma}_l) = \left(2 + \sum_{j=1}^{K} \mathbf{1}\left(\hat{\gamma}_j > 0\right)\right) \ln p \qquad\qquad (9)$$

expression dans laquelle la fonction $\mathbf{1}(.)$ est égale à 1 si son argument est vrai et zéro sinon et $\hat{\sigma}^2 = \sigma(\hat{x}_l)^2$.

Ainsi, les modèles candidats $\hat{\gamma}_l$ ayant été calculés en maximisant le critère de vraisemblance selon la relation (5), ils maximisent également le critère de vraisemblance de la relation (8). Par ailleurs, la complexité des modèles candidats $\hat{\gamma}_l$ fait intervenir le nombre des composantes de leurs vecteurs $\hat{\gamma}$ non nulles. Il est observé que la sélection à l'aide de ce type de critère est robuste et pertinente. Notamment, le modèle finalement sélectionné $\hat{\gamma}_{sel}$ est celui qui répertorie de manière pertinente les espèces $y_j$, représentée respectivement par les composantes $\hat{\gamma}_j$ du vecteur $\hat{\gamma}$.

L'étape d'analyse **12** se poursuit alors par l'exploitation, en **34,** du modèle sélectionné $\hat{\gamma}_{sel} = (\hat{\gamma}_{sel}, \gamma_{0sel})$ pour en déduire des informations sur l'échantillon biologique analysé.

Plus particulièrement, au moins une des exploitations suivantes est mise en oeuvre :

a. l'échantillon biologique est déterminé comme comprenant plusieurs espèces de référence différentes de microorganismes si le nombre de composantes du vecteur $\hat{\gamma}_{sel}$, qui sont supérieures à une valeur de seuil prédéterminée positive ou nulle, est supérieur ou égal à 2 ;

b. le nombre d'espèces de référence différentes dans l'échantillon biologique est calculé comme étant égal au nombre de composantes du vecteur $\hat{\gamma}_{sel}$ supérieures à une valeur de seuil prédéterminée positive ou nulle;

c. une espèce de référence $y_j$ est identifiée dans l'échantillon biologique lorsque la $j^{ième}$ composante du vecteur $\hat{\gamma}_{sel}$ est supérieure à une valeur de seuil positive ou nulle prédéterminée, la valeur de seuil réglant la sensibilité de l'identification ;

d. l'abondance relative $C_j$ d'une espèce $y_j$ dans l'échantillon biologique est calculée selon la relation :

$$C_j = \frac{\hat{\gamma}_{selj}}{\sum_{i=1}^{K} \hat{\gamma}_{seli}} \qquad\qquad (10)$$

où $\hat{\gamma}_{selj}$ est la $j^{ième}$ composante du vecteur $\hat{\gamma}_{sel}$ ;

e. les résultats concernant des espèces de référence appartenant à un même niveau taxonomique supérieur, notamment le genre de celles-ci, sont regroupés en calculant un scalaire $\hat{\gamma}_s^{sup}$ égal à la somme des composantes du vecteur $\hat{\gamma}_{sel}$ correspondant auxdites espèces. Le niveau taxonomique supérieur est ensuite identifié si le scalaire $\hat{\gamma}_s^{sup}$ est supérieure à la valeur de seuil prédéterminée positive ou nulle. Notamment, la $j^{ième}$ composante du vecteur $\hat{\gamma}_{sel}$ correspondant à chacune des espèces appartenant au niveau supérieur peut être inférieure à la valeur de seuil, auquel cas les espèces ne sont pas identifiés dans le mélange, alors même qu'il existe en réalité dans l'échantillon biologique au moins une espèce appartenant au niveau supérieur. Par exemple, lorsque dans un même genre regroupant des espèces de références $y_1$, $y_2$, $y_3$ difficilement différentiables par la spectrométrie de masse, la présence uniquement de l'espèce de référence particulière $y_1$ dans l'échantillon biologique peut conduire, non pas à un vecteur $\hat{\gamma}_{sel}$ dont la composante correspondant à l'espèce $y_1$ est non nulle et dont les composantes correspondant aux espèces $y_2$ et $y_3$ sont nulles, mais à un vecteur $\hat{\gamma}_{sel}$ dont les composantes correspondant aux espèces $y_1$, $y_2$, $y_3$ sont toutes non nulles mais inférieures à la valeur de seuil. En sommant les composantes de ces espèces, il est obtenu une valeur qui dépasse le seuil et par conséquent une détection du genre dans le mélange biologique. En variante, ou de manière supplémentaire, en calculant le scalaire $\hat{\gamma}_s^{sup}$ pour le niveau supérieur, l'abondance relative du niveau supérieur est calculée selon la relation $C_{sup} = \frac{\hat{\gamma}_s^{sup}}{\sum_{i=1}^{K} \hat{\gamma}_{seli}}$.

Le regroupement est avantageusement réalisé automatiquement, notamment lorsqu'il est observé que les espèces d'un même niveau taxonomique, par exemple les espèces appartenant à un même genre, sont très similaires en termes de spectre de masse. Notamment, la similarité des espèces est calculée, par exemple au moyen de coefficients de Jaccard de leur vecteur d'intensité de référence, et si la similarité calculée est supérieure à une valeur seuil, alors le regroupement des résultats des espèces est automatiquement mis en oeuvre.

Les résultats de l'exploitation sont alors mémorisés dans une mémoire informatique, par exemple celle du dispositif d'analyse et/ou affichés sur un écran à destination de l'utilisateur.

Il a été décrit un mode de réalisation particulier de l'invention. De nombreuses variantes sont cependant possibles, notamment les variantes suivantes considérées seules ou en combinaison.

Selon une variante, les modèles candidats $\hat{\gamma}_l$ ne comprennent pas de terme $\hat{\gamma}_0 I_P$ lors de la sélection par l'approche LASSO. La relation (1) se réécrit alors :

$$\hat{x}_l = \sum_{j=1}^{K} \hat{\gamma}_j P_j^{(a)} \qquad \qquad (1\text{bis})$$

Selon une variante, les modèles candidats recalculés $\hat{\gamma}_l$ lors de l'étape **30**, c'est-à-dire ceux utilisés pour la sélection du modèle final $\hat{\gamma}_{sel}$, ne comprennent pas de termes $\hat{\gamma}_0 I_P$. Les relations (4) à (11) se déduisent aisément de cette simplification. Notamment, il convient de noter que la relation (10) se réécrit selon la relation :

$$C_c(\hat{\gamma}_l) = \left( 1 + \sum_{j=1}^{K} \mathbf{1}\left(\hat{\gamma}_j > 0\right) \right) \ln p \qquad \qquad (9\text{bis})$$

Selon une variante, les coefficients $a_{ij}$ valent 1 lorsque $i = j$ et valent 0 lorsque $i \neq j$, auquel cas la relation (1) se réduit à la relation :

$$\hat{x}_l = \sum_{j=1}^{K} \hat{\gamma}_j P_j + \hat{\gamma}_0 I_P \qquad \qquad (1\text{ter})$$

Selon une variante, le coefficient de similarité $a_{ij}$ entre deux vecteurs d'intensité de référence $P_i$ et $P_j$ est le produit scalaire de ceux-ci.

Selon une variante, la sélection des structures $\tilde{\gamma}$ des vecteurs $\hat{\gamma}$ des modèles candidats $\hat{\gamma}_l$ est réalisée en mettant en oeuvre des algorithmes dérivés de l'approche LASSO de la relation (3), notamment un problème d'optimisation selon l'une des relations suivantes :

$$(\hat{\gamma}(\lambda), \hat{\gamma}_0(\lambda)) = \underset{\gamma \in R_+^K, \gamma_0 \in R_+}{argmin} \left( \left\| x - \left( \sum_{j=1}^{K} \gamma_j P_j^{(a)} + \gamma_0 I_P \right) \right\|^2 + \lambda |w_1 \odot \gamma|_1 \right) \qquad (3\text{bis})$$

$$(\hat{\gamma}(\lambda, \beta), \hat{\gamma}_0(\lambda, \beta)) = \underset{\gamma \in R_+^K, \gamma_0 \in R_+}{argmin} \left( \left\| x - \left( \sum_{j=1}^{K} \gamma_j P_j^{(a)} + \gamma_0 I_P \right) \right\|^2 + \lambda |\gamma|_1 + \beta |\gamma|_2 \right) \qquad (3\text{ter})$$

$$(\hat{\gamma}(\lambda,\beta),\hat{\gamma}_0(\lambda,\beta)) = \underset{\gamma \in R_+^K, \gamma_0 \in R_+}{\mathrm{argmin}} \left( \left\| x - \left( \sum_{j=1}^{K} \gamma_j P_j^{(a)} + \gamma_0 I_P \right) \right\|^2 + \lambda |w_1 \odot \gamma|_1 + \beta |w_2 \odot \gamma|_2 \right) \qquad (3q)$$

expressions dans lesquelles :

- $\beta$ est paramètre réel positif ;
- $|\,|_2$ est la norme L2; et
- $w_1$ et $w_2$ sont des vecteurs de poids prédéterminés de $R_+^K$.

Selon une variante, la sélection des structures $\tilde{\gamma}$ des vecteurs $\hat{\gamma}$ est réalisée au moyen d'un un algorithme de type *« stepwise »* simple ou structuré, tel que par exemple l'algorithme décrit dans le document « Structured, sparse regression with application to HIV drug resistance » de Daniel Percival et al,. Annals of Applied Statistics 2011, Vol. 5, No. 2A, 628-644, ou d'une approche exhaustive consistant à tester un nombre important, voire la totalité, des structures possibles pour le vecteur $\hat{\gamma}$.

Selon une variante, l'étape **30** de calcul des modèles candidats est omise, les modèles candidats étant ceux obtenus lors de l'étape **12,** cette étape de sélection étant alors une étape de calcul des modèles candidats par l'algorithme LASSO.

De même, il a été décrit des modes de réalisation dans lesquels les microorganismes sont référencés au niveau espèce.

En variante, plusieurs niveaux taxonomiques différents sont utilisés, par exemple au moins deux niveaux parmi l'espèce, la sous espèce et le genre.

En variante, d'autres types de caractérisation des microorganismes sont utilisés, notamment des phénotypes cliniques, comme par exemple le gram des bactéries.

De même, il a été décrit des modes de réalisation appliqués à la spectrométrie MALDI-TOF. D'autres types de mesure sont possibles, l'invention s'appliquant à la spectrométrie de masse, notamment la spectrométrie MALDI-TOF et la spectrométrie ESI-MS, à la spectroscopie vibrationnelle, notamment la spectroscopie RAMAN, à la spectroscopie par fluorescence, notamment la spectroscopie par fluorescence intrinsèque, et à la spectroscopie infrarouge.

Il va à présent être décrit des résultats d'analyse d'échantillons biologiques obtenus selon l'invention. Plus particulièrement, il est considéré une application à la spectrométrie MALDI-TOF. Les microorganismes sont référencés au niveau espèce, les modèles candidats prennent la forme de la relation (1bis), les coefficients $a_{ij}$ sont les coefficients de Jaccard de la relation (2), la sélection des structures $\tilde{\gamma}$ des vecteurs $\hat{\gamma}$ est réalisée au moyen de l'algorithme LASSO de la relation (3) en posant $\gamma_0 = \hat{\gamma}_0 = 0$, le calcul des modèles candidats est réalisé au moyen des relations (4bis), (5) et (6) avec un $\hat{\gamma}_0$ non forcé à 0, et la sélection du modèle candidat $\hat{\gamma}_{sel}$ est réalisée selon les relations (7), (8) et (9).

Il est considéré un ensemble de $K$ = 20 espèces de bactérie de référence $y_j$, certaines étant gram positives et d'autres étant gram négatives, appartenant à 9 genres différents, certaines espèces ayant été choisie en raison de la difficulté de les distinguer par spectrométrie de masse. Pour chaque espèce, 11 à 60 spectres de masse ont été mesurés à partir de 7 à 20 souches de l'espèce. Un ensemble de 571 spectres de masse pour 213 souches est ainsi constitué.

Le vecteur d'intensité de référence $P_j$ de chaque espèce $y_j$ est obtenu en appliquant une quantification constante entre 3000 et 7000 Thomson avec un nombre $p$ = 1300 intervalles, et pour chaque intervalle, une intensité de pic est calculée comme décrit précédemment à l'étape **18** pour obtenir le vecteur $P_j$.

Des échantillons biologiques ont été créés en mélangeant avec différents ratios deux espèces de références différentes, notamment :

- 4 ensembles d'échantillons biologiques, référencés « A », « B », « C », et « D », comprenant deux espèces appartenant au même genre ;
- 4 ensembles d'échantillons biologiques, référencés « E » et « F », comprenant deux espèces appartenant à des genres différents mais ayant le même type de gram ;
- 4 ensembles d'échantillons biologiques, référencés « G », « H », « I » et « J », comprenant deux espèces présentant des grams différents.

Plus particulièrement, pour chaque espèce de référence entrant dans la constitution d'un mélange, il est tout d'abord sélectionné deux souches différentes de l'espèce puis, pour chaque souche, il est produit un échantillon « pur » ne comprenant que la souche. Pour obtenir un ensemble d'échantillons biologiques mélangeant deux espèces, deux paires d'échantillons purs des deux espèces sont ensuite mélangés avec les ratios 1:0, 10:1, 5:1, 2:1, 1:1, 1:2, 1:5, 1:10,0:1.

Deux spectres de masse sont ensuite mesurés et numérisés pour chaque échantillon biologique produit, conduisant à une totalité de 360 spectres dont 80 correspondent à des échantillons purs. Chaque spectre de masse est traité pour obtenir un vecteur d'intensité $x$ en appliquant la quantification mise en oeuvre pour la construction des vecteurs d'intensité de référence, et en retenant le pic d'intensité maximale pour chaque intervalle de quantification.

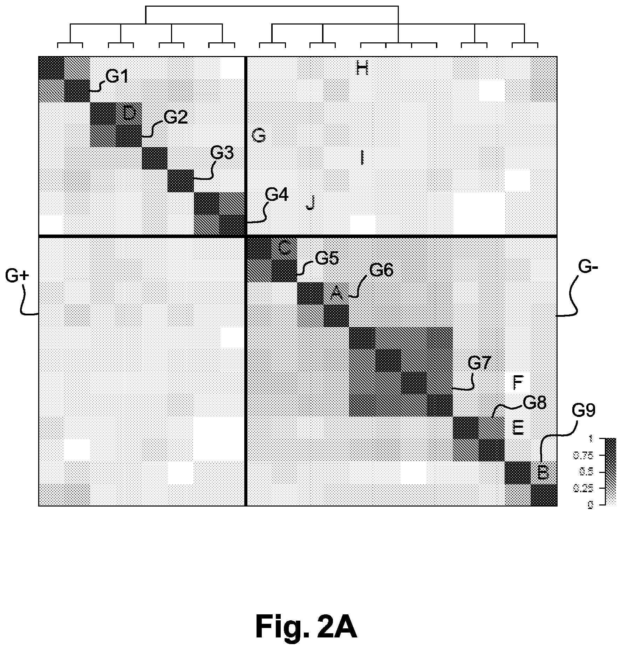

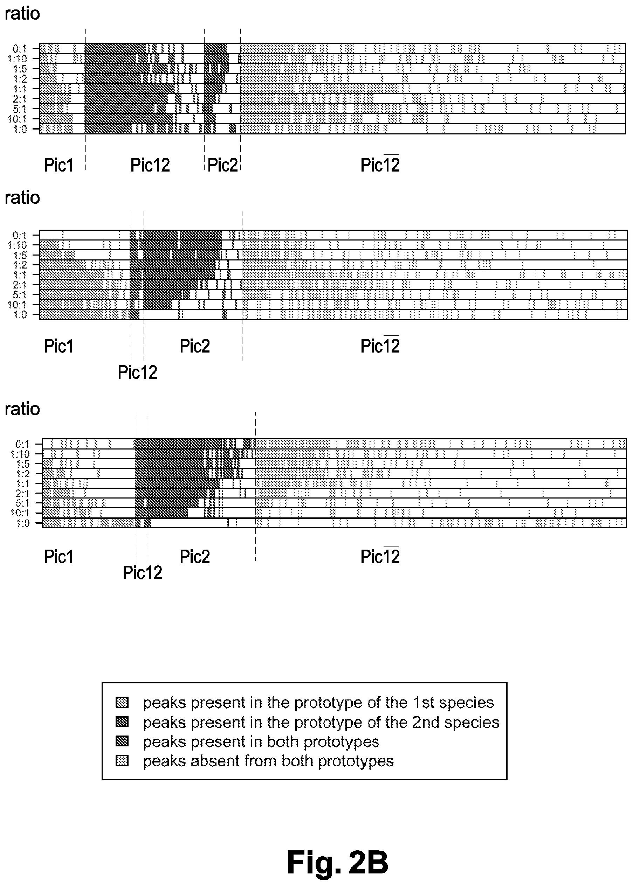

Les figures 2A et 2B offrent une illustration de l'ensemble de données de test ainsi produit. La figure 2A est une matrice de similarité des vecteurs d'intensité de référence $P_j$, les coefficients de la matrice de similarité étant des coefficients de Jaccard. Plus la composante de la matrice de similarité est foncée et plus la corrélation entre les espèces correspondantes est importante. Les carrés centraux G1-G9 correspondent aux 9 genres considérés, le carrée G+ aux bactéries gram positives et le carré G- aux bactéries gram négative. Les ensembles d'échantillon A à J sont en outre positionnés sur la matrice de similarité. La figure 2B illustre quant à elle les pics des spectres de l'ensemble D, à savoir un mélange de deux espèces appartenant au même genre, les pics des spectres de l'ensemble E, à savoir un mélange de deux espèces de bactérie ayant le même gram, et les pics des spectres de l'ensemble I, à savoir un mélange de deux espèces de bactéries de grams différents. Chaque ensemble illustré comporte neuf spectres correspondant aux différents ratios décrits ci-dessus, depuis le ratio 1:0 correspondant à un échantillon pur de la première espèce au ratio 0:1 correspondant à un échantillon pur de la seconde espèce. Les spectres illustrent notamment les pics uniquement présents dans le vecteur de référence dans la première espèce (pics « Pic1 »), les pics uniquement présents dans le vecteur de référence de la seconde espèce (pics « Pic2 »), les pics à la fois présent dans la première et la seconde espèces (pics « Pic12 »), et les pics qui ne sont présents ni dans la première espèce, ni dans la seconde espèce (pics « Pic$\overline{12}$ »). On note en particulier que pour un mélange de deux espèces de bactérie ayant le même genre, la grande majorité des pics sont présents à la fois dans les deux vecteurs de référence des deux espèces, ce qui signifie qu'il est difficile de les différencier. On remarque également que pour les mélanges de deux espèces de bactérie ayant le même gram, la proportion de pics d'une espèce présents dans le spectre du mélange évolue de manière de manière cohérente avec le ratio de cette espèce, ce qui est moins le cas pour les mélanges de deux espèces de bactérie ayant des grams différents.

La capacité du procédé selon l'invention à détecter un mélange polymicrobien et à identifier ses composants a été évaluée au moyen d'un critère de sensibilité (« sensitivity ») et d'un critère de spécificité (« specificity ») du procédé, c'est-à-dire respectivement la capacité du procédé à détecter un mélange de deux espèces et un mélange « pur ». En outre, les critères suivants sont également évalués : a) la détection d'un mélange microbien est considérée comme réussie lorsque deux ou plus de composantes sont détectées ; b) un mélange est considéré comme correctement identifié lorsque les deux espèces composant le mélange, et seulement elles, sont identifiés ; c) un mélange est considéré comme partiellement identifié lorsque l'une des deux espèces composant le mélange est identifiée ; d) L'identification d'un mélange est considérée comme ayant échoué lorsqu'une espèce n'appartenant pas au mélange est identifiée.

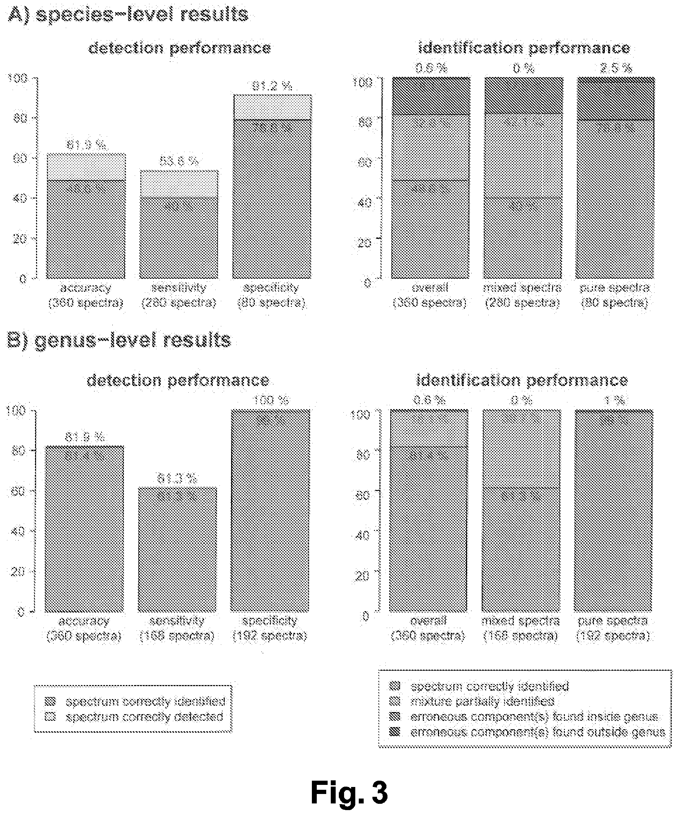

La figure 3A illustre les résultats obtenus au niveau espèce. En termes de détection (graphique de gauche), il est observé que 53,6% des mélanges polymicrobiens ont été détectés. Par contre, 91,2% des mélanges dits « purs », c'est-à-dire ne comportant qu'une seule espèce, ont été détecté et près de 75% des mélanges polymicrobiens détectés ont été correctement identifiés, ce pourcentage s'élevant à 86,4% pour les mélanges purs. En outre, concernant l'identification (graphique de droite), on note que 42,1 % des mélanges polymicrobiens ont été partiellement identifiés, conduisant à une identification partielle réussie dans 82,1% des cas, et l'identification a échouée pour environ 18% de l'ensemble des mélanges polymicrobiens et des mélanges purs. La grande majorité de ces échecs correspondent à des mélanges comprenant des espèces du même genre, ce qui est conforme à la difficulté de distinguer des bactéries qui sont proches taxonomiquement.

Le basculement de la détection et l'identification au niveau taxonomique supérieur, à savoir le genre, améliore notablement les résultats comme illustré à la figure 3B. Les résultats pour les genres ont été obtenus en mettant en oeuvre le procédé selon l'invention au niveau espèce puis en sommant les composantes des vecteurs $\hat{\gamma}_{sel}$ pour obtenir des résultats au niveau genre comme décrit précédemment. La sensibilité et la spécificité de la détection au niveau genre atteignent respectivement 61,3% et 100% pour les mélanges polymicrobiens et les mélanges purs, et tous les genres sont sensiblement correctement identifiés. En outre, les rares mélanges qui n'ont pas été correctement identifiés sont partiellement identifiés. En totalité, 81,4% des mélanges ont été correctement identifiés, l'identification ayant échouée pour seulement 0,6% d'entre eux.

**Revendications**

1. Procédé de détection dans un échantillon biologique d'au moins deux microorganismes appartenant à deux taxons différents parmi un ensemble prédéterminé $\{\hat{\gamma}_j\}$ d'un nombre de $K$ taxons de référence $y_j$ différents, chaque taxon de référence $y_j$ étant représenté par un vecteur d'intensité de référence prédéterminé $P_j$ d'un espace $R^p$ obtenu en soumettant au moins un échantillon biologique de référence comprenant un microorganisme présentant le taxon de référence à une technique de mesure produisant un signal numérique multidimensionnel représentatif de l'échantillon de référence et en déterminant ledit vecteur de référence en fonction dudit signal numérique multidimensionnel, où $p$ est supérieure à 1, le procédé comportant :

- l'acquisition d'un signal numérique multidimensionnel de l'échantillon biologique par la technologie de mesure;
- la détermination d'un vecteur d'intensité $x$ de $R^p$ en fonction du signal numérique multidimensionnel acquis ;
- la construction d'un ensemble $\{\hat{\gamma_l}\}$ de modèles candidats $\hat{\gamma_l} = (\hat{\gamma}, \hat{\gamma_0})_l$ modélisant le vecteur d'intensité $x$ selon la relation :

$$\hat{x}_l = \sum_{j=1}^{K} \hat{\gamma}_j P_j^{(a)} + \hat{\gamma}_0 I_P$$

expression dans laquelle :

- $\hat{x}_l$ est un vecteur de $R^p$ reconstruisant le vecteur d'intensité $x$ par le modèle $\hat{\gamma}_l$,
- $\hat{\gamma}_0$ scalaire est un réel et $I_P$ est le vecteur unité de $R^p$;

- $\forall j \in [[1,K]]$, $\hat{\gamma}_j$ est la $j^{\text{ième}}$ composante d'un vecteur $\hat{\gamma}$ de $R_+^K$ ;
- $\forall j \in [[1,K]]$, $P_j^{(a)} = \sum_{i=1}^{K} a_{ij} P_i$ ; et
- $\forall (i,j) \in [[1,K]]^2$, $a_{ij}$ est un coefficient prédéterminé;

- la sélection d'un modèle candidat $\hat{\gamma}_{sel}$ parmi l'ensemble $\{\hat{\gamma_l}\}$ des modèles candidats $\hat{\gamma}_l$, solution d'un problème selon la relation :

$$\hat{\gamma}_{sel} = \underset{\hat{\gamma}_l \in \{\hat{\gamma}_l\}}{argmin} \big( C_v(\hat{\gamma}_l) + C_c(\hat{\gamma}_l) \big)$$

expression dans laquelle :

- $C_v(\hat{\gamma}_l)$ est un critère quantifiant une erreur de reconstruction entre le vecteur d'intensité de l'échantillon biologique $x$ et la reconstruction $\hat{x}_l$ du vecteur d'intensité $x$ par un modèle candidat $\hat{\gamma}_l$ ; et
- $C_c(\hat{\gamma}_l)$ est un critère quantifiant la complexité d'un modèle candidat $\hat{\gamma}_l$;

- et la détermination de la présence dans l'échantillon biologique d'au moins deux microorganismes appartenant à des taxons différents de l'ensemble prédéterminé $\{\hat{\gamma_l}\}$ de taxons lorsqu'au moins deux composantes $\hat{\gamma}_j$ du vecteur $\hat{\gamma}$ du modèle candidat sélectionné $\hat{\gamma}_{sel}$ sont supérieures à une valeur de seuil prédéterminée strictement positive.

2. Procédé d'identification de microorganismes présents dans un échantillon biologique parmi un ensemble prédéterminé $\{\hat{\gamma_l}\}$ d'un nombre de $K$ taxons de référence différents, chaque taxon de référence $y_i$ étant représenté par un vecteur d'intensité de référence prédéterminé $P_j$ d'un espace $R^p$ obtenu en soumettant au moins un échantillon biologique de référence comprenant un microorganisme présentant le taxon de référence à une technique de mesure produisant un signal numérique multidimensionnel représentatif de l'échantillon de référence et en déterminant ledit vecteur de référence en fonction dudit signal numérique multidimensionnel, où $p$ est supérieure à 1, le procédé comportant :

- l'acquisition d'un signal numérique multidimensionnel de l'échantillon biologique par la technologie de mesure ;
- la détermination d'un vecteur d'intensité $x$ de $R^p$ en fonction du signal numérique multidimensionnel acquis;
- la construction d'un ensemble $\{\hat{\gamma_l}\}$ de modèles candidats $\hat{\gamma_l} = (\hat{\gamma}, \hat{\gamma_0})_l$ modélisant le vecteur d'intensité $x$ selon la relation :

$$\hat{x}_l = \sum_{j=1}^{K} \hat{\gamma}_j P_j^{(a)} + \hat{\gamma}_0 I_P$$

expression dans laquelle :

- $\hat{x}_l$ est un vecteur de $R^p$ reconstruisant le vecteur d'intensité $x$ par le modèle $\hat{\gamma}_l$;
- $\hat{\gamma}_0$ est un scalaire réel et $I_P$ est le vecteur unité de $R^p$;
- $\forall j \in [[1,K]]$, est la j$^{\text{ème}}$ composante d'un vecteur $\hat{\gamma}$ de $R_+^K$ ;
- $\forall j \in [[1,K]]$, $P_j^{(a)} = \sum_{i=1}^{K} a_{ij} P_i$ ; et
- $\forall (i,j) \in [[1,K]]^2$, $a_{ij}$ est un coefficient prédéterminé ;

    ■ la sélection d'un modèle candidat $\hat{x}_{sel}$ parmi l'ensemble $\{\hat{x}_l\}$ des modèles candidats $\hat{x}_l$, solution d'un problème selon la relation :

$$\hat{x}_{sel} = \underset{\hat{x}_l \in \{\hat{x}_l\}}{argmin}\big(C_v(\hat{x}_l) + C_c(\hat{x}_l)\big)$$

expression dans laquelle :

- $C_v(\hat{x}_l)$ est un critère quantifiant une erreur de reconstruction entre le vecteur d'intensité de l'échantillon biologique $x$ et un modèle candidat $\hat{x}_l$ ; et
- $C_c(\hat{x}_l)$ est un critère quantifiant la complexité d'un modèle candidat $\hat{x}_l$;

    ■ et la détermination de la présence dans l'échantillon biologique d'un microorganisme de taxon $y_j$ de l'ensemble prédéterminé $\{\hat{\gamma}_j\}$ pour chaque composante $\hat{\gamma}_j$ du vecteur $\hat{\gamma}$ du modèle candidat sélectionné supérieure à une valeur de seuil prédéterminée strictement positive.

**3.** Procédé de détermination de l'abondance relative dans un échantillon biologique d'au moins deux microorganismes appartenant à deux taxons différents parmi un ensemble prédéterminé $\{y_j\}$ d'un nombre de $K$ taxons de référence $y_j$ différents, chaque taxon de référence $y_j$ étant représenté par un vecteur d'intensité de référence prédéterminé $P_j$ d'un espace $R^p$ obtenu en soumettant au moins un échantillon biologique de référence comprenant un microorganisme présentant le taxon de référence à une technique de mesure produisant un signal numérique multidimensionnel représentatif de l'échantillon de référence et en déterminant ledit vecteur de référence en fonction dudit signal numérique multidimensionnel, où $p$ est supérieure à 1, le procédé comportant :

    ■ l'acquisition d'un signal numérique multidimensionnel de l'échantillon biologique par la technologie de mesure;
    ■ la détermination d'un vecteur d'intensité $x$ de $R^p$ en fonction du signal numérique multidimensionnel acquis ;
    ■ la construction d'un ensemble $\{\hat{\gamma}_l\}$ de modèles candidats $\hat{\gamma}_l = (\hat{\gamma}, \hat{\gamma}_0)_l$ modélisant le vecteur d'intensité $x$ selon la relation :

$$\hat{x}_l = \sum_{j=1}^{K} \hat{\gamma}_j P_j^{(a)} + \hat{\gamma}_0 I_P$$

expression dans laquelle :

- $\hat{x}_l$ est un vecteur de $R^p$ reconstruisant le vecteur d'intensité $x$ par le modèle $\hat{\gamma}_l$;
- $\hat{\gamma}_0$ scalaire est un réel et $I_P$ est le vecteur unité de $R^p$;
- $\forall j \in [[1,K]]$, $\hat{\gamma}_j$ est la j$^{\text{ème}}$ composante d'un vecteur $\hat{\gamma}$ de $R_+^K$ ;
- $\forall j \in [[1,K]]$, $P_j^{(a)} = \sum_{i=1}^{K} a_{ij} P_i$ ; et

◦ $\forall (i,j) \in [[1,K]]^2$, $a_{ij}$ est un coefficient prédéterminé;

▪ la sélection d'un modèle candidat $\hat{\gamma}_{sel}$ parmi l'ensemble $\{\hat{\gamma}_l\}$ des modèles candidats $\hat{\gamma}_l$, solution d'un problème selon la relation :

$$\hat{\gamma}_{sel} = \underset{\hat{\gamma}_l \in \{\hat{\gamma}_l\}}{argmin}\big(C_v(\hat{\gamma}_l) + C_c(\hat{\gamma}_l)\big)$$

expression dans laquelle :

◦ $C_v(\hat{\gamma}_l)$ est un critère quantifiant une erreur de reconstruction entre le vecteur d'intensité de l'échantillon biologique $x$ et la reconstruction $\hat{x}_l$ du vecteur d'intensité $x$ par un modèle candidat $\hat{\gamma}_l$ ; et
◦ $C_c(\hat{\gamma}_l)$ est un critère quantifiant la complexité d'un modèle candidat $\hat{\gamma}_l$;

▪ et la détermination de l'abondance relative dans l'échantillon biologique relative $C_j$ d'un taxon de référence $y_j$ selon la relation :

$$C = J(\hat{\gamma}_{sel})$$

expression dans laquelle $J$ est une fonction matricielle de $R_+^p \times R_+^K$ dans $R_+^K$ et $C = (C_1 \ldots C_j \ldots C_K)^T$ est un vecteur de $R_+^K$ avec $\forall j \in [[1,K]]$, $C_j$ est l'abondance relative du taxon de référence $y_j$.

4. Procédé selon la revendication 1, 2 ou 3, dans lequel $\forall (i,j) \in [[1,K]]^2$, $a_{ij}$ est un coefficient de similarité entre les vecteurs de référence $P_i$ et $P_j$ des taxons de référence $y_i$ et $y_j$.

5. Procédé selon la revendication 4, dans lequel le coefficient de similarité $a_{ij}$ entre les vecteurs de référence $P_i$ et $P_j$ est égal au coefficient de Jaccard entre des versions binarisées des vecteurs $P_i$ et $P_j$.

6. Procédé selon l'une quelconque des revendications précédentes, dans lequel $\hat{\gamma}_0 = 0$, et dans lequel la construction de l'ensemble $\{\hat{\gamma}_l\}$ des modèles candidats $\hat{\gamma}_l = (\hat{\gamma},0)_l$ comporte la résolution d'un ensemble de problèmes d'optimisation pour des valeurs d'un paramètre $\lambda$ de $R_+$, chaque problème étant défini selon la relation :

$$\hat{\gamma}(\lambda) = \underset{\gamma \in R_+^K}{argmin}\left(\left\|x - \sum_{j=1}^K \gamma_j P_j^{(a)}\right\|^2 + \lambda|\gamma|_1\right)$$

expression dans laquelle $|\gamma|_1$, est la norme L1 du vecteur $\gamma$.

7. Procédé selon l'une quelconque des revendications 1 à 5, dans lequel $\hat{\gamma}_0 = 0$, et dans lequel la construction de l'ensemble $\{\hat{\gamma}_l\}$ des modèles candidats $\hat{\gamma}_l = (\hat{\gamma},0)_l$ comporte la résolution d'un ensemble de problèmes d'optimisation pour des valeurs de paramètres $\lambda$ et $\beta$ de $R_+$, chaque problème étant défini selon la relation :

$$\hat{\gamma}(\lambda,\beta) = \underset{\gamma \in R_+^K}{argmin}\left(\left\|x - \sum_{j=1}^K \gamma_j P_j^{(a)}\right\|^2 + \lambda|w_1 \odot \gamma|_1 + \beta|w_2 \odot \gamma|_2\right)$$

expression dans laquelle :

▪ $|\ |_1$ est la norme L1;

- $| \, |_2$ est la norme L2;
- $a \odot b$ est le produit terme à terme des vecteurs a et b ; et
- $w_1$ et $w_2$ sont des vecteurs de poids prédéterminés de $R_+^K$.

**8.** Procédé selon l'une des revendications 6 ou 7, dans lequel pour chaque vecteur $\hat{\gamma}$ solution d'un problème d'optimisation, un nouveau modèle candidat $\hat{\gamma}_l = \left( \hat{\gamma}^{lm}, \hat{\gamma}_0^{lm} \right)_l$ est calculé, et remplace le modèle $\hat{\gamma}_l = (\hat{\gamma}, 0)_l$ correspondant au vecteur $\hat{\gamma}$, les composantes du vecteur $\hat{\gamma}^{lm}$ du nouveau modèle $\hat{\gamma}_l = \left( \hat{\gamma}^{lm}, \hat{\gamma}_0^{lm} \right)_l$, correspondant aux composantes nulles du vecteur $\hat{\gamma}$, étant forcées à zéro, et le nouveau modèle $\hat{\gamma}_l = \left( \bar{\gamma}^{lm}, \hat{\gamma}_0^{lm} \right)_l$ étant calculé en résolvant le problème d'optimisation selon les relations :

$$\left( \hat{\gamma}^{lm}, \hat{\gamma}_0^{lm} \right) = \underset{\substack{\gamma_0^{lm} \in R_+ \\ \gamma^{lm} \in R_+^K}}{\mathrm{argmax}} \left( -\frac{p}{2} ln(2\pi\sigma(x_l)^2) - \frac{1}{2\sigma(x_l)^2} \sum_{b=1}^{p} (x_b - x_{lb})^2 \right)$$

$$\sigma(x_l)^2 = \frac{1}{p} \sum_{b=1}^{p} (x_b - x_{lb})^2$$

$$x_l = \gamma_0^{lm} I_p + \sum_{j:\hat{\gamma}_j \neq 0} \gamma_j^{lm} P_j^{(a)}$$

expression dans laquelle :

- $x_b$ est la b$^{\text{ième}}$ composante du vecteur d'intensité de l'échantillon biologique $x$ ; et
- $x_{lb}$ est la b$^{\text{ième}}$ composante du vecteur de reconstruction $x_l = \gamma_0^{lm} I_p + \sum_{j:\hat{\gamma}_j > 0} \gamma_j^{lm} P_j^{(a)}$.

**9.** Procédé selon l'une quelconque des revendications précédentes, dans lequel le critère $C_v(\hat{\gamma}_l)$ quantifiant l'erreur de reconstruction est un critère de vraisemblance.

**10.** Procédé selon la revendication 9, dans lequel :

$$C_v(\hat{\gamma}_l) = -\frac{p}{2} ln(2\pi\hat{\sigma}^2) - \frac{1}{2\hat{\sigma}^2} \sum_{b=1}^{p} (x_b - \hat{x}_{lb})^2$$

expression dans laquelle :

- $\hat{\sigma}^2 = \frac{1}{p} \sum_{b=1}^{p} (x_b - \hat{x}_{lb})^2$ ;
- $x_b$ est la b$^{\text{ième}}$ composante du vecteur de pics de l'échantillon biologique $x$ ; et
- $\hat{x}_{lb}$ est la b$^{\text{ième}}$ composante du vecteur de reconstruction $\hat{x}_l$ du modèle candidat $\hat{\gamma}_l$.

**11.** Procédé selon l'une quelconque des revendications précédentes, dans lequel le critère $C_c(\hat{\gamma}_l)$ quantifiant la complexité du modèle $\hat{\gamma}_l$ quantifie ladite complexité en termes de nombre de composantes $\hat{\gamma}_j$ du vecteur $\hat{\gamma}$ strictement positives.

**12.** Procédé selon la revendication 11, dans lequel :

$$\text{Si } \hat{\gamma}_0 = 0 \text{ alors} \qquad C_c(\hat{\gamma}_l) = \left( 1 + \sum_{j=1}^{K} \mathbf{1}\left(\hat{\gamma}_j > 0\right) \right) \ln p$$

$$\text{Si } \hat{\gamma}_0 \neq 0 \text{ alors} \qquad C_c(\hat{\gamma}_l) = \left( 2 + \sum_{j=1}^{K} \mathbf{1}\left(\hat{\gamma}_j > 0\right) \right) \ln p$$

expression dans laquelle la fonction **1**(.) est égale à 1 si son argument est vrai et zéro sinon.

**13.** Procédé selon l'une quelconque des revendications précédentes, dans lequel les taxons appartiennent à un même niveau taxonomique, notamment le niveau espèce, genre ou sous-espèce.

**14.** Procédé selon l'une quelconque des revendications 1 à 12, dans lequel les taxons appartiennent à au moins deux niveaux taxonomiques différents, notamment des espèces, des genres, et/ou des sous-espèces.

**15.** Procédé selon l'une quelconque des revendications précédentes, dans lequel des taxons appartiennent à un premier niveau taxonomique, et dans lequel un modèle du vecteur *x* est calculé pour un deuxième niveau taxonomique supérieur au premier niveau taxonomique en sommant les composantes du vecteur $\hat{\gamma}$ correspondant aux taxons dépendant dudit niveau taxonomique supérieur.

**16.** Procédé selon la revendication 15, dans lequel le modèle du vecteur *x* est calculé pour le niveau taxonomique supérieur si un degré de similarité au sein du premier niveau est supérieur à un seuil prédéterminé.

**17.** Procédé selon l'une quelconque des revendications 1 à 14, dans lequel si un degré de similarité entre un ensemble de taxons définis au sein d'un premier niveau taxonomique est supérieur à un seuil prédéterminé, alors pour la constitution de l'ensemble prédéterminé $\{\hat{\gamma}_j\}$ des taxons de référence, lesdits taxons sont regroupés et remplacés par un taxon de référence défini à un deuxième niveau taxonomique, supérieur au premier niveau taxonomique.

**18.** Procédé selon l'une quelconque des revendications précédentes, dans lequel la technique de mesure produit un spectre et dans lequel les vecteurs d'intensité de référence $P_j$ sont des listes de pics compris dans les spectres des taxons de référence $y_j$.

**19.** Procédé selon la revendication 18, dans lequel la technique de mesure comprend une spectrométrie de masse.

**20.** Procédé selon l'une quelconque des revendications 3 à 19, dans lequel :

$$C_j = \frac{\hat{\gamma}_{j,sel}}{\sum_{i=1}^{K} \hat{\gamma}_{i,sel}}$$

expression dans laquelle $\forall j \in [[1,K]]$, $\hat{\gamma}_{j,sel}$ est la $j^{\text{ième}}$ composante du vecteur $\hat{\gamma}$ du modèle sélectionné $\hat{\gamma}_{sel}$.

**21.** Dispositif d'analyse d'un échantillon biologique comprenant :

- un spectromètre ou un spectroscope apte à produire des spectres de l'échantillon biologique ;
- une unité de calcul apte à mettre en oeuvre un procédé selon l'une quelconque des revendications précédentes.

**Patentansprüche**

**1.** Verfahren zur Erkennung, in einer biologischen Probe, von mindestens zwei Mikroorganismen, die zu zwei unterschiedlichen Taxa in einer vorbestimmten Gruppe $\{\gamma_j\}$ einer Anzahl von K unterschiedlichen Bezugstaxa $\gamma_j$ gehören,

wobei jedes Bezugstaxon $\gamma_j$ durch einen vorbestimmten Bezugsintensitätsvektor $P_j$ eines Raums $R^p$ dargestellt wird, der erhalten wird, indem mindestens eine biologische Bezugsprobe, die einen das Bezugstaxon aufweisenden Mikroorganismus enthält, einer Messtechnik unterzogen wird, die ein für die Bezugsprobe repräsentatives multidimensionales digitales Signal erzeugt, und indem der Bezugsvektor abhängig vom multidimensionalen digitalen Signal bestimmt wird, wobei *p* größer ist als 1, wobei das Verfahren aufweist:

■ die Erfassung eines multidimensionalen digitalen Signals der biologischen Probe durch die Messtechnologie;
■ die Bestimmung eines Intensitätsvektors *x* von $R^p$ abhängig vom erfassten multidimensionalen digitalen Signal;
■ die Konstruktion einer Gruppe $\{\hat{\gamma}_l\}$ von Kandidat-Modellen $\hat{\gamma}_l = (\hat{\gamma}, \hat{\gamma}_0)_l$, die den Intensitätsvektor *x* gemäß der folgenden Beziehung modellisiert:

$$\hat{x}_l = \sum_{j=1}^{K} \hat{\gamma}_j P_j^{(a)} + \hat{\gamma}_0 I_p$$

Ausdruck, in dem:

○ $\hat{x}_l$ ein Vektor von $R^p$ ist, der den Intensitätsvektor *x* durch das Modell $\hat{\gamma}_l$ rekonstruiert;
○ $\hat{\gamma}_0$ ein reelles Skalar und $I_p$ der Einheitsvektor von $R^p$ ist;
○ $\forall j \in [[1,K]]$, $\hat{\gamma}_j$ die j-te Komponente eines Vektors $\hat{\gamma}$ von $R_+^K$ ist;
○ $\forall j \in [[1,K]]$, $P_j^{(a)} = \sum_{i=1}^{K} a_{ij} P_i$; und
○ $\forall (i,j) \in [[1,K]]^2$, $a_{ij}$ ein vorbestimmter Koeffizient ist;

■ die Auswahl eines Kandidat-Modells $\hat{\gamma}_{sel}$ aus der Gruppe $\{\hat{\gamma}_l\}$ der Kandidat-Modelle $\hat{\gamma}_l$, Lösung eines Problems gemäß der Beziehung:

$$\hat{\gamma}_{sel} = \underset{\hat{\gamma}_l \in \{\hat{\gamma}_l\}}{argmin}\left(C_v(\hat{\gamma}_l) + C_c(\hat{\gamma}_l)\right)$$

Ausdruck, in dem:

○ $C_v(\hat{\gamma}_l)$ ein Kriterium ist, das einen Rekonstruktionsfehler zwischen dem Intensitätsvektor der biologischen Probe *x* und der Rekonstruktion $\hat{x}_l$ des Intensitätsvektors *x* durch ein Kandidat-Modell $\hat{\gamma}_l$ quantifiziert; und
○ $C_c(\hat{\gamma}_l)$ ein Kriterium ist, das die Komplexität eines Kandidat-Modells $\hat{\gamma}_l$ quantifiziert;

■ und die Bestimmung des Vorhandenseins in der biologischen Probe von mindestens zwei Mikroorganismen, die zu unterschiedlichen Taxa der vorbestimmten Gruppe $\{\gamma_j\}$ von Taxa gehören, wenn mindestens zwei Komponenten $\hat{\gamma}_j$ des Vektors $\hat{\gamma}$ des ausgewählten Kandidat-Modells $\hat{\gamma}_{sel}$ größer als ein strikt positiver vorbestimmter Schwellwert sind.

2. Verfahren zur Identifizierung von Mikroorganismen, die in einer biologischen Probe vorhanden sind, aus einer vorbestimmten Gruppe $\{\gamma_j\}$ einer Anzahl von K unterschiedlichen Bezugstaxa $\gamma_j$, wobei jedes Bezugstaxon $\gamma_j$ von einem vorbestimmten Bezugsintensitätsvektor $P_j$ eines Raums $R^p$ dargestellt wird, der erhalten wird, indem mindestens eine biologische Bezugsprobe, die einen das Bezugstaxon aufweisenden Mikroorganismus enthält, einer Messtechnik unterzogen wird, die ein für die Bezugsprobe repräsentatives multidimensionales digitales Signal erzeugt, und indem der Bezugsvektor abhängig vom multidimensionalen digitalen Signal bestimmt wird, wobei *p* größer ist als 1, wobei das Verfahren aufweist:

■ die Erfassung eines multidimensionalen digitalen Signals der biologischen Probe durch die Messtechnologie;
■ die Bestimmung eines Intensitätsvektors *x* von $R^p$ abhängig vom erfassten multidimensionalen digitalen Signal;
■ die Konstruktion einer Gruppe $\{\hat{\gamma}_l\}$ von Kandidat-Modellen $\hat{\gamma}_l = (\hat{\gamma}, \hat{\gamma}_0)_l$, die den Intensitätsvektor *x* gemäß der folgenden Beziehung modellisiert:

$$\hat{x}_l = \sum_{j=1}^{K} \hat{\gamma}_j P_j^{(a)} + \hat{\gamma}_0 I_p$$

Ausdruck, in dem:

- $\hat{x}_l$ ein Vektor von $R^p$ ist, der den Intensitätsvektor $x$ durch das Modell $\hat{\gamma}_j$ rekonstruiert;
- $\hat{\gamma}_0$ ein reelles Skalar und $I_p$ der Einheitsvektor von $R^p$ ist;

- $\forall j \in [[1,K]]$, $\hat{\gamma}_j$ die j-te Komponente eines Vektors $\hat{\gamma}$ von $R_+^K$ ist;

- $\forall j \in [[1,K]]$, $P_j^{(a)} = \sum_{i=1}^{K} a_{ij} P_i$, und
- $\forall (i,j) \in [[1,K]]^2$, $a_{ij}$ ein vorbestimmter Koeffizient ist;

  ▪ die Auswahl eines Kandidat-Modells $\hat{x}_{sel}$ aus der Gruppe $\{\hat{x}_l\}$ der Kandidat-Modelle $\hat{x}_l$, Lösung eines Problems gemäß der Beziehung:

$$\hat{x}_{sel} = arg \min_{\hat{x}_l \in \{\hat{x}_l\}} \left( C_v(\hat{x}_l) + C_c(\hat{x}_l) \right)$$

Ausdruck, in dem:

- $C_v(\hat{x}_l)$ ein Kriterium ist, das einen Rekonstruktionsfehler zwischen dem Intensitätsvektor der biologischen Probe $x$ und einem Kandidat-Modell $\hat{x}_l$ quantifiziert; und
- $C_c(\hat{x}_l)$ ein Kriterium ist, das die Komplexität eines Kandidat-Modells $\hat{x}_l$ quantifiziert;

  ▪ und die Bestimmung des Vorhandenseins in der biologischen Probe eines Mikroorganismus eines Taxons $\gamma_j$ der vorbestimmten Gruppe $\{\gamma_j\}$ für jede Komponente $\hat{\gamma}_j$ des Vektors $\hat{\gamma}$ des ausgewählten Kandidat-Modells höher als ein strikt positiver vorbestimmter Schwellwert.

3. Verfahren zur Bestimmung der relativen Abundanz in einer biologischen Probe von mindestens zwei Mikroorganismen, die zu zwei verschiedenen Taxa aus einer vorbestimmten Gruppe $\{\gamma_j\}$ einer Anzahl K von verschiedenen Bezugstaxa $\gamma_j$ gehören, wobei jedes Bezugstaxon $\gamma_j$ durch einen vorbestimmten Bezugsintensitätsvektor $P_j$ eines Raums $R^p$ dargestellt wird, der erhalten wird, indem mindestens eine biologische Bezugsprobe, die einen das Bezugstaxon aufweisenden Mikroorganismus enthält, einer Messtechnik unterzogen wird, die ein für die Bezugsprobe repräsentatives multidimensionales digitales Signal erzeugt, und indem der Bezugsvektor abhängig vom multidimensionalen digitalen Signal bestimmt wird, wobei $p$ größer ist als 1, wobei das Verfahren aufweist:

  ▪ die Erfassung eines multidimensionalen digitalen Signals der biologischen Probe durch die Messtechnologie;
  ▪ die Bestimmung eines Intensitätsvektors $x$ von $R^p$ abhängig vom erfassten multidimensionalen digitalen Signal;
  ▪ die Konstruktion einer Gruppe $\{\hat{\gamma}_l\}$ von Kandidat-Modellen $\hat{\gamma}_l = (\hat{\gamma}, \hat{\gamma}_0)_l$, die den Intensitätsvektor $x$ gemäß der folgenden Beziehung modellisiert:

$$\hat{x}_l = \sum_{j=1}^{K} \hat{\gamma}_j P_j^{(a)} + \hat{\gamma}_0 I_p$$

Ausdruck, in dem:

- $\hat{x}_l$ ein Vektor von $R^p$ ist, der den Intensitätsvektor $x$ durch das Modell $\hat{\gamma}_l$ rekonstruiert;
- $\hat{\gamma}_0$ ein reelles Skalar und $I_p$ der Einheitsvektor von $R^p$ ist;

- $\forall j \in [[1,K]]$, $\hat{\gamma}_j$ die j-te Komponente eines Vektors $\hat{\gamma}$ von $R_+^K$ ist;

$\circ \ \forall j \in [[1,K]], \ P_j^{(a)} = \sum_{i=1}^{K} a_{ij} P_i,$ und

$\circ \ \forall (i,j) \in [[1,K]]^2, a_{ij}$ ein vorbestimmter Koeffizient ist;

▪ die Auswahl eines Kandidat-Modells $\hat{\gamma}_{sel}$ aus der Gruppe $\{\hat{\gamma}_l\}$ der Kandidat-Modelle $\hat{\gamma}_l$, Lösung eines Problems gemäß der Beziehung:

$$\hat{\gamma}_{sel} = \underset{\hat{\gamma}_l \in \{\hat{\gamma}_l\}}{arg\,min} \big( C_v(\hat{\gamma}_l) + C_c(\hat{\gamma}_l) \big)$$

Ausdruck, in dem:

$\circ \ C_v(\hat{\gamma}_l)$ ein Kriterium ist, das einen Rekonstruktionsfehler zwischen dem Intensitätsvektor der biologischen Probe $x$ und der Rekonstruktion $\hat{x}_l$ des Intensitätsvektors $x$ durch ein Kandidat-Modell $\hat{\gamma}_l$ quantifiziert; und
$\circ \ C_c(\hat{\gamma}_l)$ ein Kriterium ist, das die Komplexität eines Kandidat-Modells $\hat{\gamma}_l$ quantifiziert;

▪ und die Bestimmung der relativen Abundanz in der relativen biologischen Probe $C_j$ eines Bezugstaxons $\gamma_j$ gemäß der Beziehung:

$$C = J\big( \hat{\gamma}_{sel} \big)$$

Ausdruck, in dem J eine Matrixfunktion von $R_+^p x R_+^K$ in $R_+^K$ und C = $(C_1 \ ... \ C_j \ ... \ C_K)^T$ ein Vektor von $R_+^K$ mit $\forall j \in [[1,K]]$, $C_j$ die relative Abundanz des Bezugstaxons $\hat{\gamma}_l$ ist.

4. Verfahren nach Anspruch 1, 2 oder 3, wobei $\forall (i,j) \in [[1,K]]^2$, $a_{ij}$ ein Ähnlichkeitskoeffizient zwischen den Bezugsvektoren $P_i$ und $P_j$ der Bezugstaxa $\gamma_i$ und $\gamma_j$ ist.

5. Verfahren nach Anspruch 4, wobei der Ähnlichkeitskoeffizient $a_{ij}$ zwischen den Bezugsvektoren $P_i$ und $P_j$ gleich dem Jaccard-Koeffizient zwischen binarisierten. Versionen der Vektoren $P_i$ und $P_j$ ist.

6. Verfahren nach einem der vorhergehenden Ansprüche, wobei $\hat{\gamma}_0 = 0$, und wobei die Konstruktion der Gruppe $\{\hat{\gamma}_l\}$ der Kandidat-Modelle $\hat{\gamma}_l = (\hat{\gamma},0)_l$ die Auflösung einer Gruppe von Optimierungsproblemen für Werte eines Parameters $\lambda$ von $R_+$ ist, wobei jedes Problem gemäß der folgenden Beziehung definiert wird:

$$\hat{\gamma}(\lambda) = arg\,\underset{y \in R_+^K}{min} \left( \left\| x - \sum_{j=1}^{K} \gamma_j P_j^{(a)} \right\|^2 + \lambda |\gamma|_1 \right)$$

Ausdruck, in dem $|\gamma|_1$ die Norm L1 des Vektors $\gamma$ ist.

7. Verfahren nach einem der Ansprüche 1 bis 5, wobei $\hat{y}_0 = 0$, und wobei die Konstruktion der Gruppe $\{\hat{\gamma}_l\}$ der Kandidat-Modelle $\hat{\gamma}_l = (\hat{\gamma},0)_l$ die Auflösung einer Gruppe von Optimierungsproblemen für Parameterwerte $\lambda$ und $\beta$ von $R_+$ aufweist, wobei jedes Problem gemäß der folgenden Beziehung definiert wird:

$$\hat{\gamma}(\lambda) = arg \min_{\gamma \in R_+^K} \left( \left\| x - \sum_{j=1}^{K} \gamma_j P_j^{(a)} \right\|^2 + \lambda |w_1 \odot \gamma|_1 + \beta |w_2 \odot \gamma|_2 \right)$$

Ausdruck, in dem :

- $| \ |_1$ die Norm L1 ist;
- $| \ |_2$ die Norm L2 ist;
- $\alpha \odot b$ das Produkt Term für Term der Vektoren a und b ist; und
- $w_1$ und $w_2$ vorbestimmte Gewichtsvektoren von $R_+^K$ sind.

8. Verfahren nach einem der Ansprüche 6 oder 7, wobei für jeden Vektor $\hat{\gamma}$ Lösung eines Optimierungsproblems ein neues Kandidat-Modell $\hat{\gamma}_l = \left( \hat{\gamma}^{lm}, \hat{\gamma}_0^{lm} \right)_l$ berechnet wird und das Modell $\hat{\gamma}_l = (\hat{\gamma}, 0)_l$ ersetzt, das dem Vektor $\hat{\gamma}$ entspricht, wobei die Komponenten des Vektors $\hat{\gamma}^{lm}$ des neuen Modells $\hat{\gamma}_l = \left( \hat{\gamma}^{lm}, \hat{\gamma}_0^{lm} \right)_l$, die den Komponenten Null des Vektors $\hat{\gamma}$ entsprechen, auf Null gezwungen werden, und das neue Modell $\hat{\gamma}_l = \left( \hat{\gamma}^{lm}, \hat{\gamma}_0^{lm} \right)_l$ berechnet wird, indem das Optimierungsproblem gemäß den folgenden Beziehungen aufgelöst wird:

$$\left( \hat{\gamma}^{lm}, \hat{\gamma}_0^{lm} \right) = \operatorname*{argmax}_{\substack{\gamma_0^{lm} \in R_+ \\ \gamma^{lm} \in R_+^K}} \left( -\frac{p}{2} \ln(2\pi\sigma(x_l)^2) - \frac{1}{2\sigma(x_l)^2} \sum_{b=1}^{p} (x_b - x_{lb})^2 \right)$$

$$\sigma(x_l)^2 = \frac{1}{p} \sum_{b=1}^{p} (x_b - x_{lb})^2$$

$$x_1 = y_0^{lm} I_p + \sum_{j:\hat{\gamma}_j \neq 0} y_j^{lm} P_j^{(a)}$$

Ausdruck, in dem:

- $x_b$ die b-te Komponente des Intensitätsvektors der biologischen Probe x ist; und
- $x_{lb}$ die b-te Komponente des Rekonstruktionsvektors $x_l = \gamma_0^{lm} I_p + \sum_{j:\hat{\gamma}_j > 0} \gamma_j^{lm} P_j^{(a)}$ ist.

9. Verfahren nach einem der vorhergehenden Ansprüche, wobei das den Rekonstruktionsfehler quantifizierende Kriterium $C_v(\hat{\gamma}_l)$ ein Wahrscheinlichkeitskriterium ist.

10. Verfahren nach Anspruch 9, wobei:

$$C_v(\hat{\gamma}_l) = -\frac{p}{2} \ln(2\pi\hat{\sigma}^2) - \frac{1}{2\hat{\sigma}^2} \sum_{b=1}^{p} (x_b - \hat{x}_{lb})^2$$

Ausdruck, in dem:

$$\hat{\sigma}^2 = \frac{1}{p} \sum_{b=1}^{p} (x_b - \hat{x}_{lb})^2$$

- $x_b$ die b-te Komponente des Vektors von Peaks der biologische Probe *x* ist; und
- $\hat{x}_{lb}$ die b-te Komponente des Rekonstruktionsvektors $\hat{x}_l$ des Kandidat-Modells $\hat{\gamma}_l$ ist.

11. Verfahren nach einem der vorhergehenden Ansprüche, wobei das Kriterium $C_c(\hat{\gamma})$, das die Komplexität des Modells $\hat{\gamma}$ quantifiziert, die Komplexität in Bezug auf die Anzahl von strikt positiven Komponenten $\hat{\gamma}_j$ des Vektors $\hat{\gamma}$ quantifiziert.

12. Verfahren nach Anspruch 11, wobei:

$$\text{wenn } \hat{\gamma}_0 = 0 \text{ dann}$$

$$C_c(\hat{\gamma}_l) = \left(1 + \sum_{j=1}^{K} 1\left(\hat{\gamma}_j > 0\right)\right) \ln p$$

$$\text{wenn } \hat{\gamma}_0 \neq 0 \text{ dann}$$

$$C_c(\hat{\gamma}_l) = \left(2 + \sum_{j=1}^{K} 1\left(\hat{\gamma}_j > 0\right)\right) \ln p$$

Ausdruck, in dem die Funktion 1(.) gleich 1 ist, wenn ihr Argument wahr ist, und sonst Null.

13. Verfahren nach einem der vorhergehenden Ansprüche, wobei die Taxa zum gleichen taxonomischen Niveau gehören, insbesondere dem Niveau Spezies, Gattung oder Unterspezies.

14. Verfahren nach einem der Ansprüche 1 bis 12, wobei die Taxa zu mindestens zwei unterschiedlichen taxonomischen Niveaus gehören, insbesondere Spezies, Gattungen oder Unterspezies.

15. Verfahren nach einem der vorhergehenden Ansprüche, wobei Taxa zu einem ersten taxonomischen Niveau gehören, und wobei ein Modell des Vektors *x* für ein zweites taxonomisches Niveau höher als das erste taxonomische Niveau berechnet wird, indem die Komponenten des Vektors $\hat{\gamma}$ entsprechend den Taxa summiert werden, die vom höheren taxonomischen Niveau abhängen.

16. Verfahren nach Anspruch 15, wobei das Modell des Vektors *x* für das höhere taxonomische Niveau berechnet wird, wenn ein Ähnlichkeitsgrad innerhalb des ersten Niveaus höher als eine vorbestimmte Schwelle ist.

17. Verfahren nach einem der Ansprüche 1 bis 14, wobei, wenn ein Ähnlichkeitsgrad zwischen einer Gruppe von definierten Taxa innerhalb eines ersten taxonomischen Niveaus größer als eine vorbestimmte Schwelle ist, für die Bildung der vorbestimmten Gruppe $\{\gamma_j\}$ der Bezugstaxa die Taxa neu geordnet und durch ein definiertes Bezugstaxon auf einem zweiten taxonomischen Niveau ersetzt werden, das höher ist als das erste taxonomische Niveau.

18. Verfahren nach einem der vorhergehenden Ansprüche, wobei die Messtechnik ein Spektrum erzeugt und wobei die Bezugsintensitätsvektoren $P_j$ Listen von Peaks sind, die in den Spektren der Bezugstaxa $\gamma_j$ enthalten sind.

19. Verfahren nach Anspruch 18, wobei die Messtechnik eine Massenspektrometrie enthält.

**20.** Verfahren nach einem der Ansprüche 3 bis 19, wobei:

$$C_j = \frac{\hat{\gamma}_{j,sel}}{\sum_{i=1}^{K} \hat{\gamma}_{i,sel}}$$

Ausdruck, in dem $\forall j \in [[1,K]]$, $\hat{\gamma}_{j,sel}$ die j-te Komponente des Vektors $\hat{\gamma}$ des ausgewählten Modells $\hat{\gamma}_{sel}$ ist.

**21.** Analysevorrichtung einer biologischen Probe, die enthält:

- ein Spektrometer oder ein Spektroskop, das Spektren der biologischen Probe erzeugen kann;
- eine Recheneinheit, die ein Verfahren nach einem der vorhergehenden Ansprüche anwenden kann.

**Claims**

**1.** A method of detecting in a biological sample at least two microorganisms belonging to two different taxa from a predetermined set $\{y_j\}$ of a number of $K$ different reference taxa $y_j$, each reference taxon $y_j$ being represented by a predetermined intensity vector $P_j$ of a space $R^p$ obtained by submitting at least one reference biological sample comprising a microorganism exhibiting the reference taxon to a measurement technique generating a multidimensional digital signal representative of the reference sample and by determining said reference vector according to said multidimensional digital signal, where $p$ is greater than 1, the method comprising:

- acquiring a multidimensional digital signal of the biological sample with the measurement technology;
- determining an intensity vector $x$ of $R^p$ according to the acquired multidimensional digital signal;
- constructing a set $\{\hat{\gamma}_l\}$ of candidate models $\hat{\gamma}_l = (\hat{\gamma}, \hat{\gamma}_0)_l$ modeling intensity vector x according to relation:

$$\hat{x}_l = \sum_{j=1}^{K} \hat{\gamma}_j P_j^{(a)} + \hat{\gamma}_0 I_P$$

in which expression:

- $\hat{x}_l$ is a vector of $R^p$ reconstructing intensity vector $x$ with model $\hat{\gamma}_l$;
- $\hat{\gamma}_0$ is a real scalar and $I_p$ is the unit vector of ;
- $\forall j \in [[1,K]]$, $\hat{\gamma}_j$ is the j$^{\text{th}}$ component of a vector $\hat{\gamma}$ of $R_+^K$ ;
- $\forall j \in [[1,K]]$, $P_j^{(a)} = \sum_{i=1}^{K} a_{ij} P_i$ ; and
- $\forall (i,j) \in [[1,K]]^2$, $a_{ij}$ is a predetermined coefficient;

- selecting a candidate model $\hat{\gamma}_{sel}$ from set $\{\hat{\gamma}_l\}$ of candidate models $\hat{\gamma}_l$, solution of a problem according to relation:

$$\hat{\gamma}_{sel} = \underset{\hat{\gamma}_l \in \{\hat{\gamma}_l\}}{argmin} \left( C_v(\hat{\gamma}_l) + C_c(\hat{\gamma}_l) \right)$$

in which expression:

- $C_v(\hat{\gamma}_l)$ is a criterion quantifying a reconstruction error between the intensity vector of biological sample $x$ and reconstruction $\hat{x}_l$ of intensity vector $x$ by a candidate model $\hat{\gamma}_l$; and
- $C_c(\hat{\gamma}_l)$ is a criterion quantifying the complexity of a candidate model $\hat{\gamma}_l$;

- and determining the presence in the biological sample of at least two microorganisms belonging to different taxa of predetermined set $\{y_j\}$ of taxa when at least two components $\hat{\gamma}_j$ of vector $\hat{\gamma}$ of the selected candidate

model $\hat{\gamma}_{sel}$ are greater than a strictly positive predetermined threshold value.

2. A method of identifying microorganisms present in a biological sample from a predetermined set $\{y_j\}$ of a number of $K$ different reference taxa, each reference taxon $y_j$ being represented by a predetermined intensity vector $P_j$ of a space obtained by submitting at least one reference biological sample comprising a microorganism exhibiting the reference taxon to a measurement technique generating a multidimensional digital signal representative of the reference sample and by determining said reference vector according to said multidimensional digital signal, where $p$ is greater than 1, the method comprising:

- acquiring a multidimensional digital signal of the biological sample with the measurement technology;
- determining an intensity vector $x$ of $R^p$ according to the acquired multidimensional digital signal;
- constructing a set $\{\hat{\gamma}_l\}$ of candidate models $\hat{\gamma}_l = (\hat{\gamma}, \hat{\gamma}_0)_l$ modeling intensity vector $x$ according to relation:

$$\hat{x}_l = \sum_{j=1}^{K} \hat{\gamma}_j P_j^{(a)} + \hat{\gamma}_0 I_p$$

in which expression:

- ○ $\hat{x}_l$ is a vector of $R^p$ reconstructing intensity vector $x$ with model $\hat{\gamma}_l$;
- ○ $\hat{\gamma}_0$ is a real scalar and $I_p$ is the unit vector of $R^p$;
- ○ $\forall j \in [[1,K]]$, $\hat{\gamma}_j$ is the j$^{th}$ component of a vector $\hat{\gamma}$ of $R_+^K$;
- ○ $\forall j \in [[1,K]]$, $P_j^{(a)} = \sum_{i=1}^{K} a_{ij} P_i$; and
- ○ $\forall (i,j) \in [[1,K]]^2$, $a_{ij}$ is a predetermined coefficient;

- selecting a candidate model $\hat{x}_{sel}$ from set $\{\hat{x}_l\}$ of candidate models $\hat{x}_l$, solution of a problem according to relation:

$$\hat{x}_{sel} = \underset{\hat{x}_l \in \{\hat{x}_l\}}{argmin} \left( C_v(\hat{x}_l) + C_c(\hat{x}_l) \right)$$

in which expression:

- ○ $C_v(\hat{x}_l)$ is a criterion quantifying a reconstruction error between the intensity vector of biological sample $x$ and a candidate model $\hat{x}_l$; and
- ○ $C_c(\hat{x}_l)$ is a criterion quantifying the complexity of a candidate model $\hat{x}_l$;

- and determining the presence in the biological sample of a microorganism of taxon $y_j$ of the predetermined set $\{y_j\}$ for each component $\hat{\gamma}_j$ of vector $\hat{\gamma}$ of the selected candidate model greater than a strictly positive predetermined threshold value.

3. A method of detecting the relative abundance in a biological sample at least two microorganisms belonging to two different taxa from a predetermined set $\{y_j\}$ of a number of $K$ different reference taxa $y_j$, each reference taxon $y_j$ being represented by a predetermined intensity vector $P_j$ of a space $R^p$ obtained by submitting at least one reference biological sample comprising a microorganism exhibiting the reference taxon to a measurement technique generating a multidimensional digital signal representative of the reference sample and by determining said reference vector according to said multidimensional digital signal, where $p$ is greater than 1, the method comprising:

- acquiring a multidimensional digital signal of the biological sample with the measurement technology;
- determining an intensity vector $x$ of $R^p$ according to the acquired multidimensional digital signal;
- constructing a set $\{\hat{\gamma}_l\}$ of candidate models $\hat{\gamma}_l = (\hat{\gamma}, \hat{\gamma}_0)_l$ modeling intensity vector $x$ according to relation:

$$\hat{x}_l = \sum_{j=1}^{K} \hat{\gamma}_j P_j^{(a)} + \hat{\gamma}_0 I_p$$

in which expression:

∘ $x_l$ is a vector of $R^p$ reconstructing intensity vector $x$ with model $\hat{\gamma}_l$;

∘ $\hat{\gamma}_0$ is a real scalar and $I_p$ is the unit vector of $R^p$;

∘ $\forall j \in [[1,K]]$, $\hat{\gamma}_j$ is the j$^{th}$ component of a vector $\hat{\gamma}$ of $R_+^K$;

∘ $\forall j \in [[1,K]]$, $P_j^{(a)} = \sum_{i=1}^{K} a_{ij} P_i$; and

∘ $\forall (i,j) \in [[1,K]]^2$, $a_{ij}$ is a predetermined coefficient;

■ selecting a candidate model $\hat{\gamma}_{sel}$ from set $\{\hat{\gamma}_l\}$ of candidate models $\hat{\gamma}_l$, solution of a problem according to relation:

$$\hat{\gamma}_{sel} = \underset{\hat{\gamma}_l \in \{\hat{\gamma}_l\}}{argmin}\left(C_v(\hat{\gamma}_l) + C_c(\hat{\gamma}_l)\right)$$

in which expression:

∘ $C_v(\hat{\gamma}_l)$ is a criterion quantifying a reconstruction error between the intensity vector of biological sample $x$ and reconstruction $\hat{x}_l$ of intensity vector $x$ by a candidate model $\hat{\gamma}_l$; and

∘ $C_c(\hat{\gamma}_l)$ is a criterion quantifying the complexity of a candidate model $\hat{\gamma}_l$;

■ and determining the relative abundance in biological sample $C_j$ of a reference taxon $y_j$ according to relation:

$$C = J(\hat{\gamma}_{sel})$$

in which expression $J$ is a matrix function of $R_+^p \times R_+^K$ in $R_+^K$ and $C = (C_1 \ldots C_j \ldots C_K)^T$ is a vector of $R_+^K$ with $\forall j \in [[1,K]]$, $C_j$ is the relative abundance of reference taxon $y_j$.

4. The method of claim 1, 2, or 3, wherein $\forall (i,j) \in [[1,K]]^2$, $a_{ij}$ is a coefficient of similarity between reference vectors $P_i$ and $P_j$ of reference taxa $y_i$ and $y_j$.

5. The method of claim 4, wherein the coefficient of similarity $a_{ij}$ between reference vectors $P_i$ and $P_j$ is equal to the Jaccard coefficient between binarized versions of vectors $P_i$ and $P_j$.

6. The method of any of the foregoing claims, wherein $\hat{\gamma}_0 = 0$, and wherein the construction of set $\{\hat{\gamma}_l\}$ of candidate models $\hat{\gamma}_l = (\hat{\gamma},0)_l$ comprises solving a set of optimization problems for values of a parameter $\lambda$ of $R_+$, each problem being defined according to relation:

$$\hat{\gamma}(\lambda) = \underset{\gamma \in R_+^K}{argmin}\left(\left\|x - \sum_{j=1}^{K} \gamma_j P_j^{(a)}\right\|^2 + \lambda |\gamma|_1\right)$$

in which expression $|\gamma|_1$ is norm L1 of vector $\gamma$.

7. The method of any of claims 1 to 5, wherein $\hat{\gamma}_0 = 0$, and wherein the construction of set $\{\hat{\gamma}_l\}$ of candidate models $\hat{\gamma}_l$

$= \left(\hat{\gamma}, 0\right)_l$ comprises solving a set of optimization problems for values of parameters $\lambda$ and $\beta$ of $R_+$, each problem being defined according to relation:

$$\hat{\gamma}(\lambda,\beta) = \underset{\gamma \in R_+^K}{\mathrm{argmin}} \left( \left\| x - \sum_{j=1}^{K} \gamma_j P_j^{(a)} \right\|^2 + \lambda |w_1 \odot \gamma|_1 + \beta |w_2 \odot \gamma|_2 \right)$$

in which expression:

- $| \ |_1$ is norm L1;
- $| \ |_2$ is norm L2;
- $a \odot b$ is the term-by-term product of vectors a and b; and
- $w_1$ and $w_2$ are vectors of predetermined weight of $R_+^K$.

8. The method of any of claims 6 or 7, wherein for each vector $\hat{\gamma}$ solution of an optimization problem, a new candidate model $\hat{\gamma}_l = \left(\hat{\gamma}^{lm}, \hat{\gamma}_0^{lm}\right)_l$ is calculated, and replaces model $\hat{\gamma}_l = \left(\hat{\gamma}, 0\right)_l$ corresponding to vector $\hat{\gamma}$, the components of vector $\hat{\gamma}^{lm}$ of the new model $\hat{\gamma}_l = \left(\hat{\gamma}^{lm}, \hat{\gamma}_0^{lm}\right)_l$, corresponding to the zero components of vector $\hat{\gamma}$, being forced to zero, and the new model $\hat{\gamma}_l = \left(\hat{\gamma}^{lm}, \hat{\gamma}_0^{lm}\right)_l$ being calculated by solving the optimization problem according to relations:

$$\left(\hat{\gamma}^{lm}, \hat{\gamma}_0^{lm}\right) = \underset{\substack{\gamma_0^{lm} \in R_+ \\ \gamma^{lm} \in R_+^K}}{\mathrm{argmax}} \left( -\frac{p}{2} \ln(2\pi\sigma(x_l)^2) - \frac{1}{2\sigma(x_l)^2} \sum_{b=1}^{p} (x_b - x_{lb})^2 \right)$$

$$\sigma(x_l)^2 = \frac{1}{p} \sum_{b=1}^{p} (x_b - x_{lb})^2$$

$$x_l = \gamma_0^{lm} I_p + \sum_{j : \hat{\gamma}_j = 0} \gamma_j^{lm} P_j^{(a)}$$

in which expression:

- $x_b$ is the b$^{th}$ component of the intensity vector of biological sample $x$; and
- $x_{lb}$ is the b$^{th}$ component of reconstruction vector $x_l = \gamma_0^{lm} I_p + \sum_{j : \hat{\gamma}_j > 0} \gamma_j^{lm} P_j^{(a)}$.

9. The method of any of the foregoing claims, wherein the criterion $C_v(\hat{\gamma}_l)$ quantifying the reconstruction error is a likelihood criterion.

10. The method of claim 9,-wherein:

$$C_v(\hat{\gamma}_l) = -\frac{p}{2}\ln(2\pi\hat{\sigma}^2) - \frac{1}{2\hat{\sigma}^2}\sum_{b=1}^{p}(x_b - \hat{x}_{lb})^2$$

in which expression:

$$\hat{\sigma}^2 = \frac{1}{p}\sum_{b=1}^{p}(x_b - \hat{x}_{lb})^2 \; ;$$

- $x_b$ is the b$^{th}$ component of the peak vector of biological sample $x$; and
- $\hat{x}_{lb}$ is the b$^{th}$ component of reconstruction vector $\hat{x}_l$ of candidate model $\hat{\gamma}_l$.

11. The method of any of the foregoing claims, wherein criterion $C_c(\hat{\gamma}_l)$ quantifying the complexity of model $\hat{\gamma}_l$ quantifies said complexity in terms of number of strictly positive components $\hat{\gamma}_j$ of vector $\hat{\gamma}$.

12. The method of claim 11, wherein:

$$\text{If } \hat{\gamma}_0 = 0 \text{ then} \qquad C_c(\hat{\gamma}_l) = \left(1 + \sum_{j=1}^{K} 1\left(\hat{\gamma}_j > 0\right)\right)\ln p$$

$$\text{If } \hat{\gamma}_0 \neq 0 \text{ then} \qquad C_c(\hat{\gamma}_l) = \left(2 + \sum_{j=1}^{K} 1\left(\hat{\gamma}_j > 0\right)\right)\ln p$$

in which expression function $1(.)$ is equal to 1 if its argument is true and zero otherwise.

13. The method of any of the foregoing claims, wherein the taxa belong to a same taxonomic level, particularly the species, genus, or sub-species level.

14. The method of any of claims 1 to 12, wherein the taxa belong to at least two different taxonomic level, particularly species, genera, and/or sub-species.

15. The method of any of the foregoing claims, wherein taxa belong to a first taxonomic level, and wherein a model of vector x is calculated for a second taxonomic level higher than the first taxonomic level by adding the components of vector $\hat{\gamma}$ corresponding to the taxa depending on said higher taxonomic level.

16. The method of claim 15, wherein the model of vector $x$ is calculated for the higher taxonomic level if a degree of similarity within the first level is greater than a predetermined threshold.

17. The method of any of claims 1 to 14, wherein if a degree of similarity between a set of taxa defines within a first taxonomic level is greater than a predetermined threshold, then, for the forming of the predetermined set $\{y_j\}$ of reference taxa, said taxa are gathered and replaced with a reference taxon defined at a second taxonomic level, higher than the first taxonomic level.

18. The method of any of the foregoing claims, wherein the measurement technique generates a spectrum and wherein reference intensity vectors $P_j$ are lists of peaks comprised in the spectrums of reference taxa $y_j$.

19. The method of claim 18, wherein the measurement technique comprises a mass spectrometry.

20. The method of any of claims 3 to 19, wherein:

$$C_j = \frac{\hat{\gamma}_{j,sel}}{\sum_{i=1}^{K} \hat{\gamma}_{i,sel}}$$

in which expression $\forall j \in [[1,K]]$, $\hat{\gamma}_{j\,sel}$ is the j<sup>th</sup> component of vector $\hat{\gamma}$ of selected model $\hat{\gamma}_{sel}$.

21. A device for analyzing a biological sample comprising:

- a spectrometer or a spectroscope capable of generating spectrums of the biological sample;
- a calculation unit capable of implementing the method of any of the foregoing claims.

**Fig. 1**

**Fig. 2A**

ratio

ratio

ratio

▦ peaks present in the prototype of the 1st species
▨ peaks present in the prototype of the 2nd species
▨ peaks present in both prototypes
▦ peaks absent from both prototypes

# Fig. 2B

**Fig. 3**

## RÉFÉRENCES CITÉES DANS LA DESCRIPTION

**Documents brevets cités dans la description**

- EP 2600385 A

**Littérature non-brevet citée dans la description**

- **DANIEL PERCIVAL et al.** Structured, sparse regression with application to HIV drug resistance. *Annals of Applied Statistics,* 2011, vol. 5 (2A), 628-644
- **EMILIE LABARBIER ; TRISTANT MARY-HUARD.** Le critère BIC : fondements théoriques et interprétation. *INRIA,* Septembre 2004
- **JACKSON O. LAY.** Maldi-tof spectrometry of bacteria. *Mass Spectrometry Reviews,* 2001, vol. 20, 172-194